(19) Europäisches Patentamt
European Patent Office
Office européen des brevets

(11) EP 0 433 854 B1

(12) **EUROPÄISCHE PATENTSCHRIFT**

(45) Veröffentlichungstag und Bekanntmachung des Hinweises auf die Patenterteilung:
**18.06.1997 Patentblatt 1997/25**

(21) Anmeldenummer: **90123835.2**

(22) Anmeldetag: **11.12.1990**

(51) Int Cl.6: **C12Q 1/28**, C07D 471/04, C07D 487/04, C07D 513/04 // (C07D471/04, 231:00, 221:00), (C07D487/04, 239:00, 231:00), (C07D487/04, 237:00, 231:00), (C07D513/04, 277:00, 231:00), (C07D487/04, 235:00, 231:00), (C07D487/04, 231:00, 231:00), (C07D487/04, 249:00)

(54) **3-Aminopyrazolo-Heterocyclen, deren Verwendung zur Bestimmung von Wasserstoffperoxid, Wasserstoffperoxidbildenden Systemen, Peroxidase, peroxidatisch wirksamen Substanzen oder von elektronenreichen aromatischen Verbindungen, entsprechende Bestimmungsverfahren und hierfür geeignete Mittel**

3-Aminopyrazolo heterocycles, their use for the determination of hydrogen peroxide, hydrogen peroxide forming systems, peroxidase, peroxidant active substances or electron rich aromatic compounds, corresponding determination process and agents

Hétérocycles de 3-aminopyrazole, leur utilisation pour la détermination de peroxyde d'hydrogène, systèmes formateurs de peroxyde d'hydrogène, peroxydase, substances actives comme peroxidants ou de composés aromatiques riches en électrons, procédé et agents correspondants

(84) Benannte Vertragsstaaten:
**AT BE CH DE DK ES FR GB GR IT LI LU NL SE**

(30) Priorität: **21.12.1989 DE 3942357**

(43) Veröffentlichungstag der Anmeldung:
**26.06.1991 Patentblatt 1991/26**

(73) Patentinhaber: **BOEHRINGER MANNHEIM GMBH**
**68298 Mannheim (DE)**

(72) Erfinder:
- **Zimmermann, Gerd, Dr. rer. nat.**
  **W-6800 Mannheim 41 (DE)**
- **Siedel, Joachim, Dr. rer. nat.**
  **W-8139 Bernried (DE)**
- **Frey, Günter, Chem-Ing.**
  **W-6701 Ellerstadt (DE)**

(56) Entgegenhaltungen:
**EP-A- 0 178 789**
**EP-A- 0 264 192**
**EP-A- 0 268 167**
**GB-A- 2 051 054**

- **Chemical Abstracts, Vol. 89, No. 5, 1978, Abstr. No. 43340r**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 433 854 B1

Printed by Jouve, 75001 PARIS (FR)

## Beschreibung

Die Erfindung betrifft die Verwendung von 3-Aminopyrazolo-Heterocyclen zur kolorimetrischen Bestimmung von Wasserstoffperoxid, wasserstoffperoxidbildenden Systemen, Peroxidase, peroxidatisch wirksamen Substanzen oder von elektronenreichen aromatischen Verbindungen sowie neue 3-Aminopyrazolo-Heterocyclen und ein Verfahren zu deren Herstellung.

Weiter betrifft die Erfindung ein Verfahren zur kolorimetrischen Bestimmung von Wasserstoffperoxid, Wasserstoffperoxid bildenden Systemen, Peroxidase oder peroxidatisch wirksamen Substanzen mittels oxidativer Kupplung einer elektronenreichen aromatischen Verbindung mit einer heterocyclischen Verbindung.

Im übrigen betrifft die Erfindung auch ein Mittel zur kolorimetrischen Bestimmung von Wasserstoffperoxid, Wasserstoffperoxid bildenden Systemen, Peroxidase oder peroxidatisch wirksamen Substanzen durch oxidative Kupplung enthaltend eine elektronenreiche aromatische Verbindung und eine heterocyclische Verbindung.

Desweiteren betrifft die Erfindung die Verwendung eines 3-Aminopyrazoloderivates zur Herstellung eines Mittels zur kolorimetrischen Bestimmung von Wasserstoffperoxid, Wasserstoffperoxid bildenden Systemen, Peroxidase oder peroxidatisch wirksamen Substanzen durch oxidative Kupplung.

Außerdem betrifft die Erfindung ein Verfahren zur kolorimetrischen Bestimmung einer elektronenreichen aromatischen Verbindung durch deren oxidative Kupplung mit einer heterocyclischen Verbindung in Gegenwart eines Oxidationsmittels.

Die Erfindung betrifft auch ein Mittel zur kolorimetrischen Bestimmung einer elektronenreichen aromatischen Verbindung durch oxidative Kupplung enthaltend eine heterocyclische Verbindung und ein Oxidationsmittel.

Schließlich betrifft die Erfindung die Verwendung eines 3-Aminopyrazoloderivates zur Herstellung eines Mittels zur kolorimetrischen Bestimmung einer elektronenreichen aromatischen Verbindung durch oxidative Kupplung.

Sowohl für die analytische Chemie, als auch für die medizinische Diagnostik, ist der Nachweis von Wasserstoffperoxid mittels geeigneten chromogenen Substanzen, unter Katalyse von Peroxidase oder peroxidatisch wirksamen Substanzen wie z.B. Hämoglobin, von sehr großer Bedeutung. Dies gilt vor allem für die zahlreichen Nachweisverfahren, bei denen Wasserstoffperoxid als Zwischenprodukt gebildet und im Anschluß unter Anwesenheit geeigneter chromogener Substanzen, vorwiegend in Gegenwart einer Peroxidase (POD) als Katalysator, in eine Verbindung überführt wird, die optisch erfaßbar ist und in einer quantitativen Beziehung zum gebildeten Wasserstoffperoxid steht. Weiterhin wird Peroxidase häufig als Enzym-Marker in Immuntesten verwendet und durch Zusatz von Wasserstoffperoxid und obigen chromogenen Substanzen nachgewiesen.

Als Beispiele sind nachfolgend Verbindungen genannt, die mit den in Klammer stehenden entsprechenden Oxidasen wasserstoffperoxidbildende Systeme darstellen:

Glucose (Glucoseoxidase), Galactose (Galactoseoxidase), L-Aminosäure (L-Aminosäureoxidase), Cholesterin (Cholesterinoxidase), Harnsäure (Uricase), Sarcosin (Sarcosinoxidase), Glycerin (Glycerinoxidase).

Für den Nachweis von Wasserstoffperoxid/Peroxidase sind bereits zahlreiche Chromogene bzw. Indikatorsysteme genannt und eingesetzt worden. Eines der bekanntesten ist das von Trinder (Ann. Clin. Biochem. 6 (1969), 24 - 27) beschriebene Indikatorsystem, bei dem Phenol mit 4-Aminoantipyrin in Gegenwart von POD unter Einwirkung von $H_2O_2$ oxidativ zu einem Farbstoff gekuppelt wird. Dabei können anstelle des Phenols als Kupplungskomponente auch Phenolderivate, Anilinderivate, Naphthol, Naphtholderivate, Naphthylamin, Naphthylaminderivate oder ähnlich reagierende Substanzen treten. 4-Aminoantipyrin als Kupplungspartner kann z.B. durch Aminoantipyrinderivate, Vanilindiaminsulfonsäure, Methylbenzthiazolinonhydrazon (MBTH), sulfoniertes Methylbenzthiazolinonhydrazon (SMBTH) und ähnliche von S. Hünig beschriebene Systeme (vgl. "The chemistry of Synthetic Dyes", K. Venkataraman Ed., Vol. IV S. 189-193; Academic Press, New York San Francisco, London, 1971), substituiert werden.

In EP-A-0 033 539 wird zur Erzielung besserer Farbstabilitäten der bei der oxidativen Kupplung von 4-Aminoantipyrin mit Phenolen entstehenden Farbstoffe der Ersatz von 4-Aminoantipyrin durch 4-Aminoantipyrinderivate vorgeschlagen, die einen zweifach substituierten Phenylrest tragen. Die so erzielbaren Farbstoffe weisen $\lambda_{max}$ -Werte im Bereich von etwa 500 - 550 nm auf.

Insgesamt besteht ein großer Nachteil der bisher beschriebenen Redoxindikatorsysteme auf der Basis von 4-Aminoantipyrin oder N-Methylbenzthiazolinon-hydrazon darin, daß durch oxidative Kupplung mit elektronenreichen aromatischen Verbindungen, wie Phenolen oder Anilinen, Farbstoffe entstehen, deren $\lambda_{max}$ -Werte im Bereich um 500 ± 50 nm herum liegen. Oxidative Kupplungsreaktionen werden häufig angewandt bei der Analytik von Körperflüssigkeiten, wie Blut, Plasma, Serum, Harn, Speichel etc. Insbesondere bei hämolysierten Plasmen oder Seren, d. h. hämoglobinhaltigen Plasmen oder Seren entstehen aber Probleme bei Verwendung von Redoxindikatorsystemen des Standes der Technik welche oxidative Kupplungsreaktionen eingehen, die durch die relativ hohe Eigenabsorption solchen Probenmaterials im Bereich um 500 nm bedingt sind. Beeinträchtigungen der Messung sind dann um so gravierender je geringer die Konzentration des zu bestimmenden Serumbestandteils ist.

Außerdem sind viele der bekannten Redoxindikatoren wegen der durch Autoxidation bedingten Leerwertinstabilität oder der Instabilität der erzeugten Farbe bei den für die enzymatischen Reaktionen notwendigen pH-Werten von ca.

6-8 nicht für die Entwicklung einer $H_2O_2$-Bestimmung geeignet.

Dies trifft insbesondere auch auf das von W. Ried und E.V. Köcher in Liebigs Ann. Chem. 647, 116 - 144 (1961) und Liebigs Ann. Chem. 647, 144 - 154 (1961) unter anderen beschriebene 2-Hydroxy-3-amino-5,7-dimethyl-pyrazolopyrimidin zu, das gegen Sauerstoff so unbeständig ist, daß es nicht als Reinsubstanz gewonnen werden konnte.

EP-A-0 268 167 beschreibt Pyrazolderivate, die mit einem primären aromatischen Amin für die Bestimmung von Wasserstoffperoxid verwendet werden. Der Substituent in der 3-Position des Pyrazolrings wird dabei abgespalten.

Trotz der Vielzahl bekannter Redoxindikatoren zum Nachweis von Wasserstoffperoxid, Peroxidase oder peroxidatisch wirksamer Substanzen wird immer noch nach solchen Verbindungen gesucht, die in möglichst vielen Testsystemen, insbesondere zur Bestimmung von Bestandteilen von Körperflüssigkeiten, einsetzbar sind, über einen breiten pH-Bereich eine gleichmäßige, hohe Empfindlichkeit zeigen und nicht durch Serumbestandteile gestört werden.

Im Rahmen der Aufgabe der vorliegenden Erfindung sollten deshalb geeignete Verbindungen bereitgestellt werden, die für die Bestimmung von Wasserstoffperoxid, Wasserstoffperoxid bildenden Systemen, Peroxidase, peroxidatisch wirksamen Substanzen oder von elektronenreichen aromatischen Verbindungen brauchbar sind, die durch oxidative Kupplung mit Phenolen, Anilinen oder anderen Kuppiern Farbstoffe liefern, deren Absorption genügend langwellig ist, um durch die Eigenabsorption von Blutplasma oder -serum nicht gestört zu werden, die den Anforderungen bezüglich Leerwertstabilität und Farbstabilität beim Meß-pH genügen und bei denen die Farbausbeute bei der oxidativen Kupplung nicht durch Blutbestandteile gestört wird.

Diese Aufgabe wird durch die vorliegende Erfindung, so wie sie in den Ansprüchen charakterisiert ist, gelöst.

Es wurde gefunden, daß Pyrazoloderivate der allgemeinen Formel I

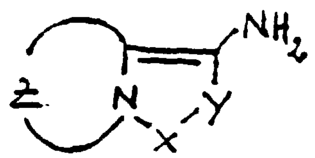

(I)

in der
X-Y $NR^1$-CO oder $N=CR^2$ bedeutet,
wobei

$R^1$ Alkyl und

$R^2$ Alkyl, Alkenyl, Alkoxy, Alkylthio, Aryl, Aralkyl, gegebenenfalls jeweils substituiert durch Hydroxy, Dialkylphosphinyl, Carboxy, $SO_3H$, $PO_3H_2$, ein Salz eines dieser Säurereste oder/und Alkoxycarbonyl;
Amino, das gegebenenfalls durch ein oder zwei gegebenenfalls einen oder mehrere Hydroxy-, Carboxy- oder/und Alkoxycarbonylreste tragende Alkylreste substituiert ist wobei, wenn Amino durch 2 Alkylreste substituiert ist, diese Reste auch zu einem Ring geschlossen sein können, der außer durch das N-Atom der Aminogruppe gegebenenfalls auch durch Sauerstoff, Schwefel oder ein weiteres Stickstoffatom unterbrochen sein kann oder Amino gegebenenfalls durch ein oder zwei Acylgruppen, Alkoxy- oder/und Aralkoxycarbonylgruppen, $H_2N$-CO, Alkyl-, Aralkyl- oder/und Arylcarbamoylgruppen substituiert ist; oder
Wasserstoff, Carboxy, Alkoxycarbonyl, Carboxamido oder Halogen ist, und

Z $NR^3$-N=N bedeutet,
wobei $R^3$ Alkyl oder Aralkyl ist oder

Z eine ungesättigte Kette mit 3 bis 5 Gliedern aus Stickstoffatomen oder aus Kohlenstoffatomen und gegebenenfalls einem oder mehreren Stickstoff- oder oder Schwefelatomen darstellt, wobei Kohlenstoffatome gegebenenfalls substituiert sind durch Alkyl, Alkoxy, Alkylthio, Hydroxy, Aralkyl, Aryl, Carboxy, Carboxamido, Alkoxycarbonyl, Cyano, Amino, das gegebenenfalls durch ein oder zwei gegebenenfalls einen oder mehrere Hydroxy-, Carboxy- oder/und Alkoxycarbonylreste tragende Alkylreste substituiert ist oder Halogen und wobei Stickstoffatome, die nicht über eine Doppelbindung gebunden sind, durch Alkyl oder Aralkyl substituiert sind oder zwei benachbarte Kettensubstituenten gegebenenfalls eine Alkylengruppe bilden, die ihrerseits gegebenenfalls mit Aryl substituiert oder anelliert ist

sowie gegebenenfalls entsprechende tautomere Formen und deren Salze
zur kolorimetrischen Bestimmung von Wasserstoffperoxid, wasserstoffperoxidbildenden Systemen, Peroxidase,

peroxidatisch wirksamen Substanzen oder elektronenreichen aromatischen Verbindungen vorteilhaft einsetzbar sind.

Hierbei bedeutet "Alkyl" - auch in Alkylthio-, Dialkylphosphinyl-, Alkylcarbamoyl- und Aralkylresten - einen geradkettigen oder verzweigten Alkylrest mit 1-6, vorzugsweise 1-4 C-Atomen. Beispiele sind die Methyl-, Ethyl-, Propyl-, iso-Butyl- oder tert.-Butylgruppe.

Wenn eine Aminogruppe durch 2 Alkylreste substituiert ist, können diese Reste auch so zu einem Ring geschlossen sein, daß sie insgesamt einen durch ein Stickstoffatom unterbrochenen Ring darstellen. Bevorzugt sind hierbei solche Aminogruppen, die einen insgesamt 5- oder 6-gliedrigen Ring darstellen und der seinerseits gegebenenfalls durch Sauerstoff, Schwefel oder Stickstoff unterbrochen ist. Besonders bevorzugt ist der Morpholinorest.

"Alkoxy" - auch in Alkoxy- und Aralkoxycarbonylresten - steht für einen geradkettigen oder verzweigten Alkoxyrest mit 1 - 6, vorzugsweise 1 - 4 C-Atomen. Beispiele sind die Methoxy-, Ethoxy-, Propyloxy-, iso-Butyloxy- oder tert.-Butyloxygruppe.

"Aryl" - auch in Arylcarbamoylgruppen - bezeichnet einen Kohlenstoffaromaten- oder Heteroaromatenrest, vorzugsweise einen solchen mit 6 - 10 Ring-Atomen, insbesondere die Phenyl- und die Naphthylgruppe, die zusätzlich noch durch. Alkyl, Alkoxy oder/und Halogen substituiert sein können. Besonders bevorzugt ist der Phenylrest.

Ein "Aralkyl"-Rest - auch in einer Aralkylcarbamoylgruppe - bedeutet einen Rest, bei dem eine wie vorstehend definierte Alkylgruppe durch einen wie zuvor charakterisierten Arylrest substituiert ist. Bevorzugt ist die Benzylgruppe.

Ein "Aralkoxy"-Rest, beispielsweise in Aralkoxycarbonylgruppen, bezeichnet einen Rest, bei dem eine wie vorstehend definierte Alkoxygruppe durch einen wie zuvor charakterisierten Arylrest substituiert ist. Bevorzugt ist die Benzyloxygruppe.

"Halogen" steht für die Reste Fluor, Chlor, Brom und Jod. Fluor und Chlor sind bevorzugt.

Eine Acylgruppe bezeichnet einen Carbonsäurerest, der Alkyl-, Aralkyl- oder Arylreste enthalten kann. Bevorzugt sind Acetyl-, Phenylacetyl- oder Benzoylreste.

Unter einer Alkylengruppe wird eine geradkettige oder verzweigte, gesättigte oder ungesättigte Kohlenwasserstoffkette aus 3 - 5, vorzugsweise 3 oder 4 C-Atomen mit zwei freien Bindungsstellen verstanden.

Beispiele sind $-CH_2-CH=CH-$,

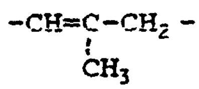, 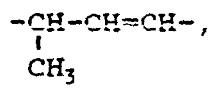,

$-(CH_2)_4-$ oder $-CH=CH-CH=CH-$.

Bevorzugt sind der Butadiendiylrest ($-CH=CH-CH=CH-$) und der Tetramethylenrest ($-(CH_2)_4-$).

Ein Alkenylrest ist ein geradkettiger oder verzweigter Kohlenwaserstoffrest aus 2-5 C-Atomen mit mindestens einer Doppelbindung. Bevorzugt ist beispielsweise der Vinylrest. Unter einer Dialkylphosphinylgruppe wird der Rest

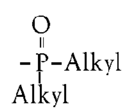

verstanden, wobei Alkyl die zuvor gegebene Bedeutung hat. Bevorzugt ist der Dimethylphosphinylrest.

Als Salze von $SO_3H$, $PO_3H_2$ und Carboxyresten können Alkali- oder Erdalkalisalze oder Ammoniumsalze eingesetzt werden. Unter Alkalisalzen werden Lithium-, Natrium-, Kalium-, Rubidium- und Cäsiumsalze verstanden, wobei Lithium-, Natrium- und Kaliumsalze, vor allem aber Natrium- und Kaliumsalze bevorzugt sind. Erdalkalisalze sind solche des Berylliums, Magnesiums, Calciums, Strontiums oder Bariums.

Bevorzugt sind Magnesium- und Calciumsalze, wobei Calciumsalze besonders bevorzugt sind. Als Ammoniumsalze können solche des unsubstituierten Ammoniumions, $NH_4^+$, Verwendung finden. Es ist aber auch möglich, solche Ammoniumsalze einzusetzen, bei denen das Ammoniumion durch 1 - 4 Alkyl-, Aryl- oder Aralkylreste substituiert ist. Für diese Reste gelten die zuvor gegebenen Definitionen, wobei als Alkylrest Methyl, Ethyl und n-Propyl, als Arylrest die Phenylgruppe und als Aralkylrest die Benzylgruppe besonders bevorzugt sind.

Als Carboxamidorest wird der Rest $CONH_2$ verstanden, aber auch solche Reste, bei denen die Aminogruppe durch ein oder zwei gegebenenfalls einen oder mehrere Hydroxy-, Carboxy- oder/und Alkoxycarbonylreste tragende Alkylreste substituiert ist.

In den erfindungsgemäß eingesetzten Pyrazoloderivaten der allgemeinen Formel I ist Z vorzugsweise so angeordnet, daß mindestens eine Doppelbindung der ungesättigten Kette in Konjugation zu der Doppelbindung oder zu dem N-Atom der allgemeinen Formel I steht.

Gegebenenfalls sind für eine Substanz der allgemeinen Formel I auch tautomere Formen möglich. Diese sollen ebenfalls als von der allgemeinen Formel I umfaßt gelten.

Die 3-Amino-pyrazoloderivate der allgemeinen Formel I können als freie Amine eingesetzt werden. Bevorzugt werden sie jedoch als entsprechende Ammoniumsalze verwendet. Hierfür sind Salze mit den verschiedensten Säuren möglich. Besonders bevorzugt sind solche Amine der allgemeinen Formel I, die mit einer Mineralsäure, wie Salzsäure, Schwefelsäure, Phosphorsäure oder Salpetersäure als Salz gebunden vorliegen. Hervorragend bewährt haben sich Hydrochloride der Substanzen der allgemeinen Formel I.

Erfindungsgemäß bevorzugt sind Substanzen der allgemeinen Formeln II bis XIII

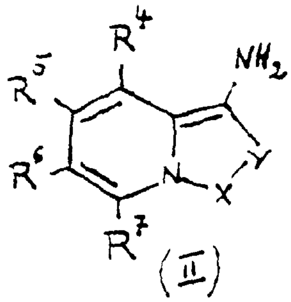
(II)

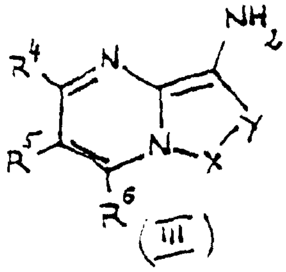
(III)

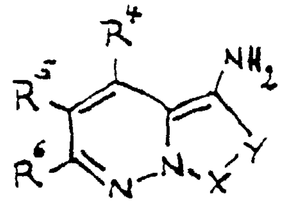
(IV)

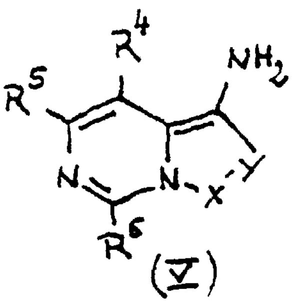
(V)

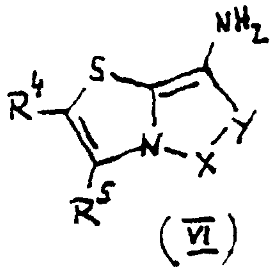
(VI)

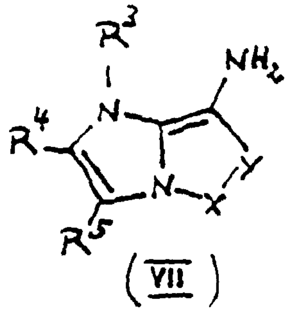
(VII)

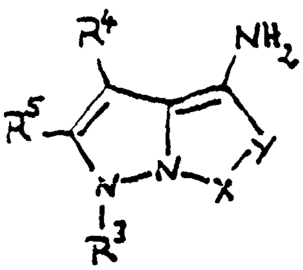
(VIII)

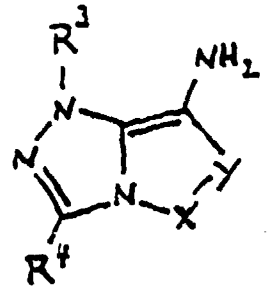
(IX)

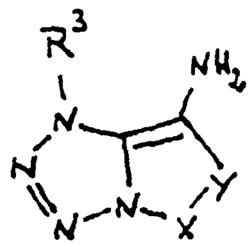
(X)

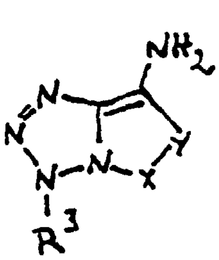
(XI)

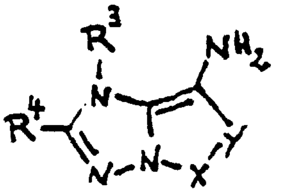
(XII)

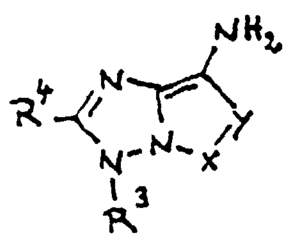
(XIII)

sowie gegebenenfalls entsprechende tautomere Formen und deren Salze.
Hierbei haben X-Y und $R^3$ die gleiche Bedeutung wie zuvor beschrieben. $R^4$, $R^5$, $R^6$ und $R^7$, die gleich oder verschieden sind, stehen für Wasserstoff, Hydroxy, Alkyl, Alkoxy, Alkylthio, Aralkyl, Aryl, Carboxy, Alkoxycarbonyl, Carboxamido, Cyano, Amino, das gegebenenfalls durch ein oder zwei gegebenenfalls einen oder mehrere Hydroxy-, Carboxy- oder/und Alkoxycarbonylreste tragende Alkylreste substituiert ist oder Halogen, wobei zwei benachbarte Reste gegebenenfalls eine Alkylengruppe bilden, die ihrerseits gegebenenfalls mit Aryl substituiert oder anelliert ist. Die Definitionen der Reste entsprechen den für die Substanzen der allgemeinen Formel I gegebenen.

Besonders bevorzugt für die erfindungsgemäße Verwendung sind Substanzen der allgemeinen Formeln II, III, IV, VI, VII und IX, gegebenenfalls entsprechende tautomere Formen und deren Salze. Ganz besonders bevorzugt sind solche Substanzen, in denen X-Y die Bedeutung $N=CR^2$ hat, wobei $R^2$ die Bedeutung haben kann wie für die allgemeine Formel I angegeben.

Als hervorragend geeignet für die erfindungsgemäße Verwendung hat sich insbesondere die Verbindung 3-Amino-2-methyl-pyrazolo-[1.5a]-pyridin und deren Salze, insbesondere das Hydrochlorid erwiesen.

Einige Verbindungen der allgemeinen Formel I sind aus dem Stand der Technik als therapeutisch wirksame Substanzen bekannt.

So werden in DE-A-22 57 547 und J. Med. Chem. 17, 645 (1974) 3-Amino-5,7-dimethylpyrazolo[1.5-a]pyrimidine als 3',5'-cyclo-AMP-Phosphodiesterase-Inhibitoren beschrieben.

In DE-A-30 19 019 werden Amino-pyrazolo[1.5-c]chinazolin-Derivate als Substanzen beschrieben, die schmerzstillende bzw. analgetische, die Magensäuresekretion hemmende und die Darmtätigkeit herabsetzende bzw. antiperistaltische Wirkungen zeigen.

In EP-A-0 299 209 werden 3-Aminopyrazolopyridine mit diuretischer, hypotensiver, vasodilatierender, cardiotonischer und die Blutplättchenaggregation inhibierender Wirkung genannt.

Aus Chemical Abstracts 89, 627, 43340r (1978) ist ein tetracyclisches 3-Aminopyrazolotriazinderivat bekannt.

Die Verwendung von Pyrazoloderivaten der allgemeinen Formel I als Komponente in oxidativen Kupplungsreaktionen zum kolorimetrischen Nachweis von Wasserstoffperoxid, Wasserstoffperoxid bildenden Systemen, Peroxidase, peroxidatisch wirksamen Substanzen oder von elektronenreichen aromatischen Verbindungen ist in dem vorgenannten Stand der Technik nicht beschrieben.

Zur erfindungsgemäßen Durchführung der kolorimetrischen Bestimmung von Wasserstoffperoxid, Wasserstoffperoxid bildenden Systemen, Peroxidase oder peroxidatisch wirksamer Substanzen mittels oxidativer Kupplung muß eine elektronenreiche aromatische Verbindung mit einem vorstehend beschriebenen Pyrazolderivat umgesetzt werden. Zur besseren Charakterisierung werden im folgenden die erfindungsgemäßen Pyrazoloderivate als Kupplungskomponente und die zur oxidativen Kupplung notwendigen elektronenreichen aromatischen Verbindungen als Kuppler bezeichnet.

Als Kuppler, der mit einer erfindungsgemäßen Kupplungskomponente eine oxidative Kupplung einzugehen vermag, kommt prinzipiell jede Substanz in Frage, die auch mit p-Phenyldendiamin als Kupplungskomponente eine solche Reaktion eingehen kann. Dies ist für den Fachmann einfach zu prüfen. Hierzu sind eine Reihe von Substanzen, beispielsweise aus der Farbfotographie bekannt. So sind die in T.H. James, "The theory of the photographic process", 3rd ed., McMillan, New York, 1966, chapter 17 ("Principles and Chemistry of Color Photography"), S. 382 - 396 genannten phenolischen Kuppler und Kuppler mit aktiven Methylengruppen geeignet, mit den erfindungsgemäßen Pyrazoloderivaten eine oxidative Kupplung einzugehen. Am gebräuchlichsten sind Phenol, Phenolderivate, Naphthol, Naphtholderivate, Naphthylamin, Naphthylaminderivate oder Anilinderivate.

Bevorzugte Kuppler im Sinne der vorliegenden Erfindung sind Anilinophosphonsäurederivate, wie sie in EP-A-0 175 250 beschrieben sind, N,N-Dimethylanilin, 2,4,6-Tribrom-3-hydroxybenzoesäure oder N-Ethyl-N-3-sulfo-2-hydroxypropyl-m-anisidin.

Die erfindungsgemäßen Nachweisreaktionen, die zur Bildung eines Farbstoffes führen, lassen sich wie folgt am Beispiel eines N,N-Dimethylanilins als Kuppler formulieren:

Schema 1:

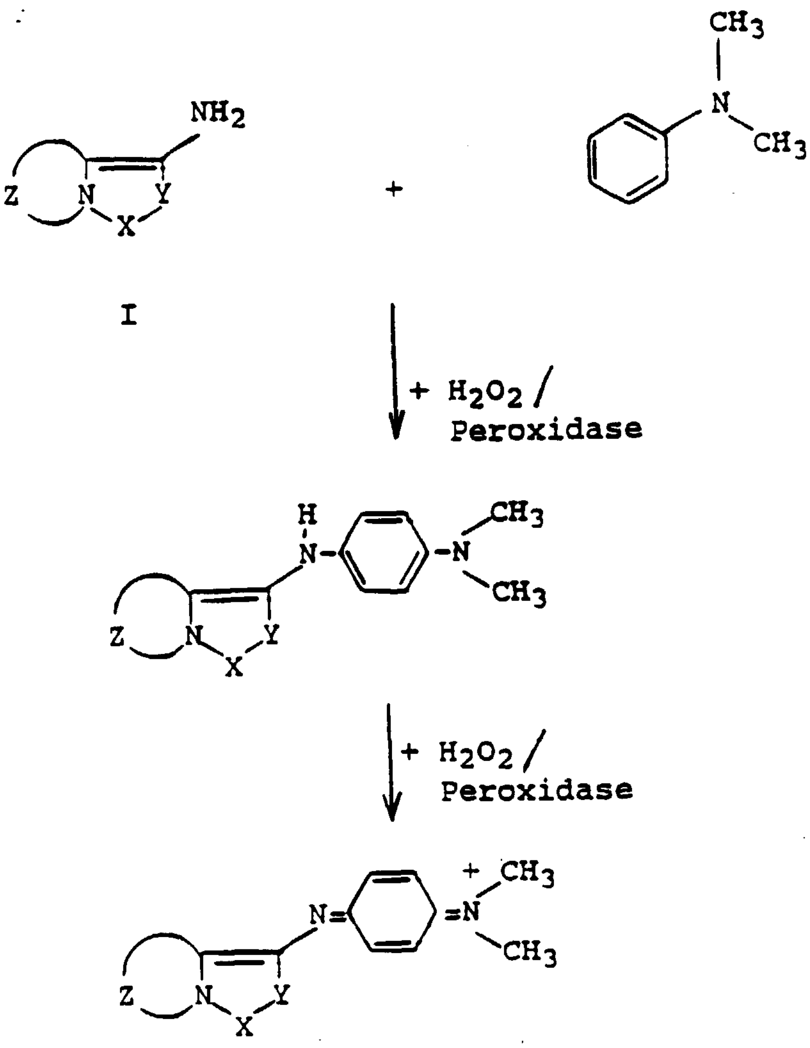


Verallgemeinernd läßt sich hieraus ersehen, daß zum Nachweis von Wasserstoffperoxid oder eines dieses liefernden Systems, ein Pyrazoloderivat der allgemeinen Formel I, ein Kuppler und Peroxidase oder eine peroxidatisch wirksame Substanz mit der zu untersuchenden Probe in Kontakt gebracht werden müssen. Unter Wasserstoffperoxid liefern-den Systemen sind insbesondere die in der klinischen Diagnostik wichtigen Substrat/Substratoxidase-Paare zu verstehen, von denen stellvertretend einige der wichtig-sten Vertreter bereits in der Einleitung vorgestellt wurden und bei denen das Substrat in Gegenwart von Luftsauerstoff oxidiert und Wasserstoffperoxid produziert wird. Es lassen sich somit entweder Substrat oder Substratoxidase nachweisen, je nachdem, welche Komponente bekannt ist und zusammen mit den übrigen Nachweisreagenzien mit der zu untersuchenden Probe kontaktiert wird.

Soll das Enzym Poroxidase oder eine peroxidatisch wirksame Substanz bestimmt werden, muß die Probe mit Kuppler, Pyrazoloderivat der allgemeinen Formel I und Wasserstoffperoxid zur Reaktion gebracht werden. Alternativ kann natürlich auch eine Substanz nachgewiesen werden, die als Kuppler, wie beispielsweise in Schema 1 N,N-Dimethylanilin, wirken kann. Es muß dann ein Pyrazoloderivat der allgemeinen Formel I und ein Oxidationsmittel, das nicht unbedingt das System Wasserstoffperoxid/Peroxidase sein muß, sondern auch ein anderes Oxidationsmittel sein kann, beispielsweise ein Peroxid, wie Persulfat oder Peracetat, oder Perjodat, Chloramin T oder insbesondere ein Cyanoferri-Komplex, beispielsweise Kaliumhexacyanoferrat (III) oder eine Oxidase gemäß DE-A-33 31 588, mit der Probe in Kontakt gebracht werden.

Die bei positivem Ergebnis auftretende Farbe kann bezüglich ihrer Intensität als Maß für die Menge der zu bestimmenden Substanz herangezogen werden. Sie kann visuell oder photometrisch ausgewertet werden.

Mit den zur Durchführung des erfindungsgemäßen Verfahrens zur kolorimetrischen Bestimmung von Wasserstoffperoxid, Wasserstoffperoxid bildenden Systemen, Peroxidase, peroxidatisch wirksamen Substanzen oder elektronenreichen aromatischen Verbindungen erforderlichen Substanzen lassen sich erfindungsgemäße Mittel herstellen, die in einer Küvette vermessen werden können. Mittel zur Bestimmung von Wasserstoffperoxid, Wasserstoffperoxid bildenden Systemen, Peroxidase oder peroxidatisch wirksamen Substanzen enthalten erfindungsgemäß eine elektronenreiche aromatische Verbindung und als oxidativ hiermit kuppelbare heterocyclische Substanz ein Pyrazoloderivat der allgemeinen Formel I. Separat oder zusammen mit diesen Substanzen können weitere für den jeweiligen Parameternachweis notwendige Stoffe, wie Peroxidase zur Bestimmung von Wasserstoffperoxid; Peroxidase und Enzymsubstrat zur Bestimmung des das Substrat unter Bildung von Wasserstoffperoxid oxidierenden Enzyms; Peroxidase und oxidierendes Enzym zur Bestimmung des durch das Enzym unter Bildung von Wasserstoffperoxid oxidierbaren Enzymsubstrats; oder Wasserstoffperoxid zur Bestimmung von Peroxidase oder peroxidatisch wirksamen Substanzen vorliegen. Mittel zur kolorimetrischen Bestimmung einer elektronenreichen aromatischen Verbindung enthalten ein Oxidationsmittel und ein Pyrazoloderivat der allgemeinen Formel I.

Weitere Bestandteile der erfindungsgemäßen Mittel können Puffer sowie gegebenenfalls Netzmittel, Aktivatoren und andere Hilfsstoffe sein.

Die Reagenzbestandteile können zusammen, getrennt oder je nach Verträglichkeit und Zweckmäßigkeit miteinander kombiniert als Pulver, zu Tabletten verpreßt oder vorzugsweise in Wasser oder Puffer gelöst und gegebenenfalls anschließend eingetrocknet oder lyophilisiert zu dem erfindungsgemäßen Mittel verarbeitet sein.

Eine so gewonnene feste Reagenzmischung wird vor Gebrauch in Wasser oder einem anderen geeigneten Lösungsmittel gelöst und so die Reagenzlösung bereitet. Nach Vermischen der Probe (beispielsweise Substratlösung, Enzymlösung, Serum oder Plasma) mit einem aliquoten Teil der Reagenzmischung wird die entstehende Farbe an einem Photometer vermessen und über den molaren Extinktionskoeffizienten und die zugesetzten Reagenz- bzw. Probevolumina die jeweilige Konzentration bzw. Substratkonzentration berechnet. Es sind sowohl kinetische als auch Endpunktmessungen möglich.

Erfindungsgemäße Mittel können die erfindungsgemäß verwendbaren Pyrazoloderivate zusammen mit den übrigen für den jeweiligen Parameternachweis notwendigen Substanzen, wie Oxidationsmittel, Enzyme, elektronenreiche aromatische Substanz oder/und Enzyymsubstrat, Puffer, gegebenenfalls Netzmittel und Aktivatoren sowie andere Hilfsstoffe auch in oder auf quellfähigen oder saugfähigen Reagenzträgern, wie Polymerfilmen, Papieren, Membranen, Vliesen etc. enthalten. Dazu kann man je nach Verträglichkeit bzw. Zweckmäßigkeit eine oder mehrere Lösungen in Form von wässrigen oder organischen bzw. gemischten Lösungen herstellen, je nachdem wie sich die Reagenzien oder Hilfsstoffe lösen. Mit diesen Lösungen werden saugfähige Glas- oder Kunststoffvliese, imprägniert oder besprüht. Anschließend wird getrocknet. Die so hergestellten Reagenzträger können entweder als Schnelldiagnostica zur direkten Bestimmung von Inhaltsstoffen von Flüssigkeiten (z.B. in Körperflüssigkeiten wie Blut, Urin oder Speichel, oder in Lebensmitteln z.B. Fruchtsäften, Milch o.a.) eingesetzt werden. Die Flüssigkeit wird dabei direkt auf den Reagenzträger gebracht oder dieser kurz in die Flüssigkeit eingetaucht. Eine semiquantitative Bestimmung ist möglich, indem man die so entstandene Farbe einer Vergleichsfarbe zuordnet. Eine quantitative Auswertung läßt sich remissionsphotometrisch durchführen. Durch Eluieren der vorweggenannten Reagenzien mit Wasser bzw. Puffer oder Serum aus dem saugfähigen Träger läßt sich eine Reagenzlösung herstellen, mit der man wie oben beschrieben, Substrate oder Enzyme in einer Küvette am Photometer bestimmen kann. Die quantitative Bestimmung durch remissionsphotometrische Auswertung läßt sich besonders gut durchführen, wenn ein erfindungsgemäß verwendbares Pyrazoloderivat gemeinsam mit den restlichen notwendigen Reagenzien und Hilfsstoffen und einem filmbildenden Kunststoff, z.B. gemäß DE-C-1598153, zu einem Reagenzfilm verarbeitet wird. Die glatte Oberfläche eines solchen Films ergibt dabei weniger Störungen der Remission und eine homogenere Färbung als die üblicherweise verwendeten saugfähigen Papiere.

Puffer im Sinne dieser Erfindung haben einen pH-Wert von 5-10, insbesondere 6,5 - 8 Phosphat-, Citrat-, Borat-, GOOD-Puffer mit Alkali- oder Ammoniumgegenionen werden am häufigsten verwendet, andere Systeme sind jedoch ebenfalls brauchbar.

Netzmittel sind insbesondere anionische und kationische Netzmittel. Nichtionogene Netzmittel, die Enzyme aktivieren, sind jedoch ebenfalls brauchbar. Natriumlaurylsulfat, Dioctylnatriumsulfosuccinat und Alkylarylpolyetheralkohole werden bevorzugt.

Als Aktivatoren sind bei der Bestimmung eines Enzyms oder Enzymsubstrats als Beispiel eines Wasserstoffperoxid bildenden Systems die für die jeweilige Enzymreaktion bekannten einzusetzen. Oft ist die farbbildende Reaktion so schnell, daß eine zusätzliche Aktivierung nicht notwendig erscheint.

Als sonstige Hilfsstoffe können übliche Verdicker, Emulgatoren, optische Aufheller, Kontrastmittel etc. sinnvoll sein, wie sie in entsprechenden Testen mit anderen Chromogenen bekannt sind.

Die Kupplungsreaktion erfolgt üblicherweise bei Raumtemperatur, kann jedoch auch ohne weiteres bei höherer Temperatur, beispielsweise bei 37°C, durchgeführt werden, wenn dies für die Reaktionsgeschwindigkeit beispielsweise einer vorgeschalteten enzymatischen Reaktion sinnvoll ist.

Für die üblicherweise vorkommenden Reaktionen mit Substraten bzw. Substrat oxidierenden Enzymen haben sich folgende Konzentrationen der Testlösung bewährt:

| | |
|---|---|
| Pyrazoloderivat | 0,05 - 50 mmol/l<br>vorzugsweise 0,1 - 1 mmol/l |
| Kuppler | 0,05 - 100 mmol/l |
| Puffer pH 5 -10<br>vorzugsweise pH 6,5 - 8 | 0,05 - 1 Mol/l<br>vorzugsweise 0,1 - 0,5 Mol/l |
| Netzmittel | 0 - 1,0 Mol/l<br>vorzugsweise 0,05 - 0,1 Mol/l |
| a) Peroxidase | 1,0 - 5000 KU/1 |
| b) $H_2O_2$ bzw. $H_2O_2$-produzierendes Substrat/Enzymgemisch 0,1 - 10 mMol/l | |
| sonstige Hilfsstoffe | 0 - 5 Mol/l |

Die vorstehenden Konzentrationsbereiche sind so zu verstehen, daß die unteren Bereiche jeweils für die photometrischen Teste in der Küvette und die oberen Bereiche für Schnellteste bzw. Teste auf festen Trägern bevorzugt sind.

Die erfindungsgemäß verwendbaren Pyrazoloderivate zeigen viele Vorteile. So ermöglichen sie aufgrund quantitativer oder nahezu quantitativer Reaktion mit entsprechenden Kupplern eine sehr hohe Farbausbeute. Die durch die oxidative Kupplung erzeugten Farbstoffe, insbesondere diejenigen, die mit Anilinderivaten gemäß EP-A-0 175 250 als Kuppler entstehen, sind stabil und weisen eine hohe Extinktion auf. Außerdem liegt das Absorptionsmaximum ($\lambda_{max}$) der Farbstoffe so hoch, daß auch hämolysiertes Plasma oder Serum als Probenmaterial verwendet werden kann. Die erfindungsgemäß verwendbaren Pyrazoloderivate zeichnen sich außerdem durch eine schnelle Farbbildung mit einem Kuppler unter oxidativen Kupplungsbedingungen aus. Darüber hinaus zeigen die erfindungsgemäß verwendbaren Pyrazoloderivate eine geringe Autoxidationsneigung, so daß sie die für einen kommerziellen Test notwendige Lagerstabilität aufweisen.

Ein weiterer Gegenstand der Erfindung sind die nicht aus dem Stand der Technik bekannten und damit neuen Pyrazoloderivate der allgemeinen Formel I'

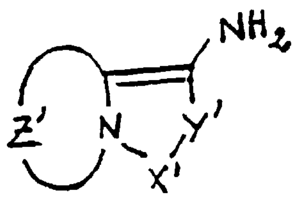

(I')

in der

X'-Y' $NR^{1'}$-CO oder $N{=}CR^{2'}$ bedeutet,
wobei $R^{1'}$ Alkyl und
$R^{2'}$ Alkyl, Alkenyl, Alkoxy, Alkylthio, Aryl, Aralkyl, gegebenenfalls jeweils substituiert durch Hydroxy, Dialkylphoshinyl, Carboxy, $SO_3H$, $PO_3H_2$, ein Salz eines dieser Säurereste oder/ und Alkoxycarbonyl;
Amino, das gegebenenfalls durch ein oder zwei gegebenenfalls einen oder mehrere Hydroxy-, Carboxy- oder/ und Alkoxycarbonylreste tragende Alkylreste substituiert ist wobei, wenn Amino durch 2 Alkylreste substituiert ist, diese Reste auch zu einem Ring geschlossen sein können, der außer durch das N-Atom der Aminogruppe gegebenenfalls auch durch Sauerstoff, Schwefel oder ein weiteres Stickstoffatom unterbrochen sein kann oder Amino gegebenenfalls durch ein oder zwei Acylgruppen, Alkoxy- oder/und Aralkoxycarbonylgruppen, $H_2N$-CO, Alkyl-, Aralkyl- oder/und Arylcarbamoylgruppen substituiert ist; oder
Wasserstoff, Carboxy, Alkoxycarbonyl, Carboxamido oder Halogen ist, und

Z' $NR^{3'}$-N=N bedeutet,
wobei $R^{3'}$ Alkyl oder Aralkyl ist oder
Z' eine ungesättigte Kette mit 3 bis 5 Gliedern aus Stickstoffatomen oder aus Kohlenstoffatomen und gegebenenfalls einem oder mehreren Stickstoff- oder Schwefelatomen darstellt, wobei sämtliche Kohlenstoffatome der ungesättigten Kette Teil einer Doppelbindung sind und wobei Kohlenstoffatome gegebenenfalls substituiert sind durch Alkyl, Alkoxy, Alkylthio, Hydroxy, Aralkyl, Aryl, Carboxy, Carboxamido, Alkoxycarbonyl, Cyano, Amino, das gegebenenfalls durch ein oder zwei gegebenenfalls einen oder mehrere Hydroxy-, Carboxy- oder/ und Alkoxycarbonylreste tragende Alkylreste substituiert ist oder Halogen und wobei Stickstoffatome, die nicht über eine Doppelbindung gebunden sind durch Alkyl oder Aralkyl substituiert sind oder zwei benachbarte Kettensubstituenten gegebenenfalls eine Alkylengruppe bilden, die ihrerseits gegebenenfalls mit Aryl substituiert oder anelliert ist,
oder gegebenenfalls entsprechende Tautomere
und deren Salze
unter der Bedingung, daß,

a) wenn X'-Y' gleich N=CR$^{2'}$ mit R$^{2'}$ gleich Alkyl oder Phenyl und
Z' eine ungesättigte Kette aus 4 Kohlenstoffatomen ist, diese nicht unsubstituiert und nicht durch Alkyl, Alkoxy oder Halogen substituiert ist,

b) wenn Z' eine ungesättigte Kette aus vier Gliedern mit 3 Kohlenstoffatomen und einem Stickstoffatom am Ende der Kette bedeutet, so daß in Formel I' der Ring mit Z' einen Pyrimidinring bildet und ein oder mehrere Kohlenstoffatome substituiert sind durch Alkyl, Alkoxy, Alkylthio, Hydroxy, Aryl, Alkoxycarbonyl oder Halogen oder mit einem Benzolring kondensiert ist und

X'-Y' für N=CR$^{2'}$ steht,
R$^{2'}$ nicht Wasserstoff bedeutet,

c) wenn Z'

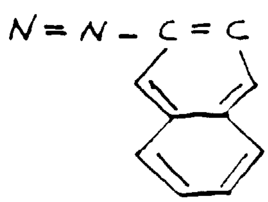

oder

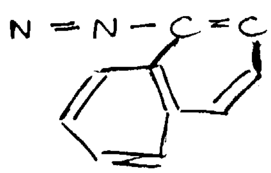

bedeutet, so daß in Formel I' der Ring mit Z' einen 1,2,4-Triazinring bildet und

X'-Y' für N=CR$^{2'}$ steht,
R$^{2'}$ nicht Methyl ist.

Die Bedeutung der einzelnen Reste entspricht der für die allgemeine Formel I angegebenen. Ebenso gelten für die neuen Pyrazoloderivate die gleichen Ausführungen wie sie für die erfindungsgemäß verwendbaren Pyrazoloderivate bezüglich vorteilhafter Reste und Gruppen gemacht sind.

Außerdem ist Gegenstand der Erfindung ein Verfahren zur Herstellung von Pyrazoloderivaten der allgemeinen Formel I, das dadurch gekennzeichnet ist, daß eine Verbindung der allgemeinen Formel XIV
in der

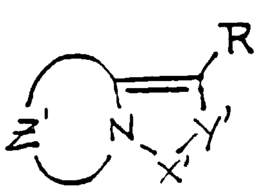

(XIV)

X'-Y' und Z' die gleiche Bedeutung wie für die allgemeine Formel I' haben und
R Wasserstoff ist,

a) durch Umsetzung mit Salpetersäure oder Salpetersäure im Gemisch mit Schwefelsäure und/oder Acetanhydrid in eine Verbindung der allgemeinen Formel XIV mit R gleich Nitro oder

b) durch Reaktion mit salpetriger Säure in eine Verbindung der algemeinen Formel XIV mit R gleich Nitroso oder

c) durch Reaktion mit einem aromatischen Diazoniumsalz in eine Verbindung der allgemeinen Formel XIV mit R gleich Arylazo

überführt und anschließend reduziert wird.

Die Synthese der Verbindungen der allgemeinen Formel I' kann nach an sich bekannten Methoden erfolgen, indem man eine Verbindung der allgemeinen Struktur XIV nach an sich bekannten Methoden in die Aminoverbindung überführt.

Nitro-, Nitroso- und Arylazoreste, das sind Reste Aryl-N=N-, wobei Aryl die gleiche Bedeutung haben kann, wie zuvor für Arylreste und andere solche Reste enthaltende Gruppen erläutert, lassen sich durch Reduktion mit Reagentien, wie Zink in Säure, beispielsweise Salzsäure oder Eisessig, Natriumdithionit, Zinn in Säure, beispielsweise Salzsäure, Zinn(II)-chlorid oder durch katalytische Hydrierung beispielsweise über Palladium/Kohle in die Aminoverbindung überführen. Solche Reaktionen sind in Houben-Weyl, Methoden der organischen Chemie, Bd. 11/1, S. 341 f beschrieben.

Die Einführung von Nitro, Nitroso oder Arylazogruppen ausgehend von Verbindungen der Formel XIV mit R = H kann durch Nitrierung mit Salpetersäure oder Salpetersäure in Gemischen mit konzentrierter Schwefelsäure oder Acetanhydrid erfolgen. Durch Nitrosierung mit salpetriger Säure oder durch Azokupplung mit aromatischen Diazoniumsalzen, lassen sich die Nitrosogruppe bzw. eine Arylazogruppe einführen. Beispiele für solche Reaktionen sind in Houben-Weyl, Methoden der organischen Chemie Bd. 10/1 und 10/3 beschrieben.

In einigen Fällen ist auch die direkte Nitrosierung von Verbindungen der Formel XIV möglich, in der R für Alkoxycarbonyl, beispielsweise Acetyl steht, ohne durch Kochen mit konzentrierter Salzsäure zuvor eine Hydrolyse zu einer Verbindung der Struktur XIV mit R = H durchführen zu müssen (vgl. Chem. Pharm. Bull. 22, 482 (1974).

Liegen Heterocyclen der allgemeinen Formel XIV mit R gleich Carboxyl, Alkoxycarbonyl oder Alkylcarbonyl vor, so können diese durch Hydrolyse mit konzentrierter Salzsäure und - bei Carbonsäuren - thermischer Decarboxylierung in die Verbindungen der allgemeinen Formel XIV mit R = H überführt werden. Daran schließt sich dann die Einführung einer Nitro-, Nitroso- oder Arylazogruppe an.

Stickstoffatome, die nicht an einer Doppelbindung stehen, und in den Resten X'-Y' oder Z' der allgemeinen Struktur I' vorkommen, müssen alkyliert oder aralkyliert sein. Die N-Alkylierung bzw. N-Aralkylierung läßt sich durch Umsetzung der entsprechenden Verbindungen der Struktur XIV in der R die oben gegebene Bedeutung hat, mit Alkylierungs-bzw. Aralkylierungsmitteln, wie Alkyl- oder Aralkylhalogeniden, Dialkyl- bzw. Diaralkylsulfaten oder Arylsulfonsäurealkylestern bzw. -aralkylestern in Gegenwart einer Base, wie Natriumhydrid, tertiären Aminen, Alkalicarbonaten oder Natriumhydroxid in Lösungsmitteln, wie Dimethylformamid oder wässrig-alkoholische Systeme durchführen.

Die benötigten Ausgangsprodukte sind beschrieben oder lassen sich analog zu bekannten Verbindungen synthetisieren. Informationen über die Herstellung der heterocyclischen Systeme sind in folgenden Publikationen enthalten: G.P. Ellis "Synthesis of fused Heterocyles", in "The Chemistry of Heterocyclic Compounds", E.C. Taylor Eds., 1987, John Wiley and Sons; P.N. Preston, "Condensed Imidazoles" in "The Chemistry of Heterocyclic Compounds", A. Weissberger und E.C. Taylor Eds., 1986, John Wiley and Sons; Adv. of Het. Chem. 36, 343 (1984); Chem. Pharm. Bull. 22, 482 (1974); J. Het. Chem. 12, 481 (1975); Chem. Pharm. Bull. 22, 1814 (1974); Ann. 660, 104 (1962); Chem. Pharm. Bull. 23, 452 (1975); J. Het. Chem. 10, 411 (1973); J. Chem. Soc. Perkin I 2047 (1977).

Die Verbindungen der allgemeinen Formel I' sind Basen, die mit organischen oder anorganischen Säuren Salze bilden, die sich vorteilhaft für die Isolierung und Reinigung verwenden lassen.

Die nachfolgenden Beispiele sollen die Erfindung weiter erläutern, ohne daß die Erfindung als darauf beschränkt verstanden werden soll.

Beispiel 1

3-Amino-2-methoxy-pyrazolo - [1,5-a]-pyridin-hydrochlorid

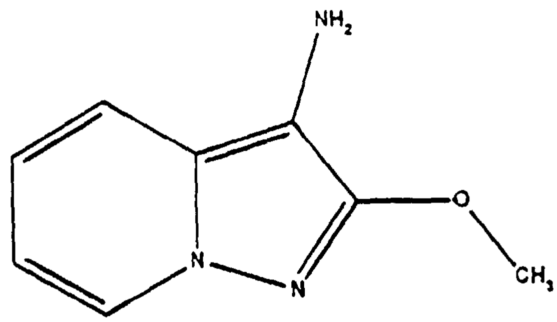

1.1 1,48 g 2-Methoxy-pyrazolo[1.5-a]-pyridin (Bull. Chem. Soc. Jap. 49, 1980 (1976)) werden unter Eiskühlung in 20 ml konzentrierter Salpetersäure gelöst und tropfenweise mit 10,5 ml rauchender Salpetersäure versetzt. Man rührt das Gemisch 30 Minuten im Eisbad und 1 Stunde bei Raumtemperatur. Die Reaktionslösung wird auf Eis gegossen, der ausgefallene Niederschlag abgesaugt und mit Wasser gewaschen. Man erhält 1 g (52 % der Theorie) 2-Methoxy-3-nitro-pyrazolo-[1.5-a]-pyridin (Fp 213-216 °C.)

1.2 0,8 g der in 1.1 erhaltenen Nitroverbindung werden in 80 ml 2 N Salzsäure suspendiert und unter heftigem Rühren mit Zinkstaub versetzt. Nach mehrmaliger Zugabe von Zinkstaub erhält man nach ca. 1 Stunde eine klare Lösung über einem Bodensatz von überschüssigem Zink. Dieser wird abfiltriert und das Filtrat mit Natriumhydroxid auf pH 7 gestellt. Die Mischung wird mit Essigsäureethylester extrahiert. Die organische Phase wird eingedampft und das verbleibende Öl mit Ethanol über eine kurze Schicht Kieselgel filtriert. Das Eluat wird eingedampft, der Rückstand in Ether gelöst und mit etherischer Salzsäure versetzt. Der ausgefallene Niederschlag wird abgesaugt und nochmals aus Isopropanol umkristallisiert. Man erhält 0,33 g (42 % der Theorie) der Titelverbindung mit Fp. 238-241 °C.
DC (Kieselgel, Aceton/Methylenchlorid/Eisessig 50:45:5): $R_F$ = 0,6.

Beispiel 2

3-Amino-2-chlor-5,7-dimethyl-pyrazolo-[1.5-a]-pyrimidinhydrochlorid

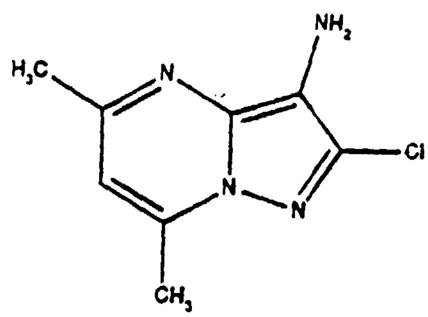

Analog Beispiel 1 erhält man ausgehend von 2-Chlor-5,7-dimethylpyrazolo-[1,5-a]-pyrimidin (Ann. Chem. 647, 116 (1961)) 3-Amino-2-chlor-5.7-dimethyl-pyrazolo-[1.5-a]-pyrimidin-hydrochlorid mit Fp.227-230 °C.
DC (Kieselgel, Aceton/Metylenchlorid/Eisessig 50:45:5): Rf = 0.8

Beispiel 3

3-Amino-2-methyl-pyrazolo-[1.5-a]-pyridin-hydrochlorid

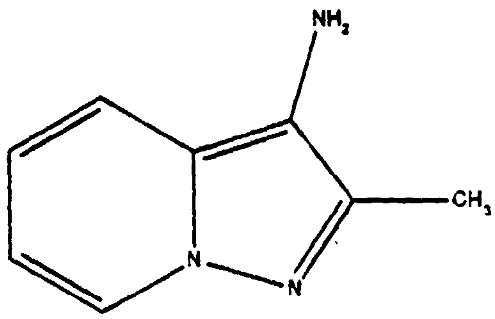

7 g 3-Acetyl-2-methyl-pyrazolo-[1.5-a-]pyridin (J. Org. Chem. 42, 443 (1977)) werden in 140 ml 6 N Salzsäure gelöst und bei 0 °C tropfenweise mit einer Lösung von 5,52 g Natriumnitrit in Wasser versetzt. Nach 2 Stunden wird das Eisbad entfernt und die Reaktionsmischung über Nacht bei Raumtemperatur stehen lassen und dann auf pH 9 gestellt. Die ausgefallene Nitrosoverbindung (6.4 g) wird abgesaugt und in ca. 150 ml 2 N Salzsäure gelöst. Analog Beispiel 1.2 wird die Nitrosoverbindung mit Zinkstaub reduziert. Das Rohprodukt wird an Kieselgel mit Essigsäureethylester chromatographiert. Die Produktfraktionen werden eingeengt, in Ethanol gelöst und mit ethanolischer Chlorwasserstoffsäure versetzt. Der nach einiger Zeit ausfallende Niederschlag wird abgesaugt und getrocknet. Man erhält 3.1 g (45 % der Theorie) der Titelverbindung mit Fp. > 275 °C, DC (Kieselgel, Essigsäureethylester/Aceton/Eisessig/Wasser 50:25:12,5:12,5): Rf = 0.6

Beispiel 4

3-Amino-2-methyl-pyrazolo-[3.2 b]-thiazol-hydrochlorid

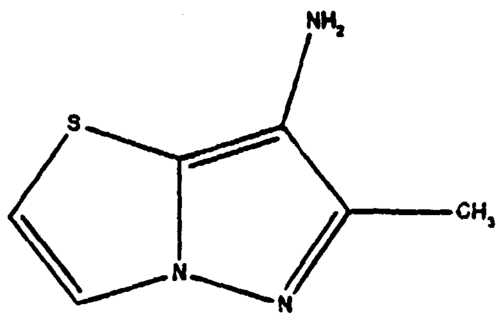

Analog Beispiel 3 erhält man aus 3-Acetyl-2-methyl-pyrazolo-[3.2-b]-thiazol (Chem. Pharm. Bull. 22, 482 (1974)) 3-Amino-2-methyl-pyrazolo-[3.2-b]-thiazol-hydrochlorid mit Fp 215-218 °C, DC (Kieselgel, Methanol): Rf = 0,65

Beispiel 5

3-Amino-2-methoxy-5,7-dimethyl-pyrazolo-[1,5-a]-pyrimidin-hydrochlorid

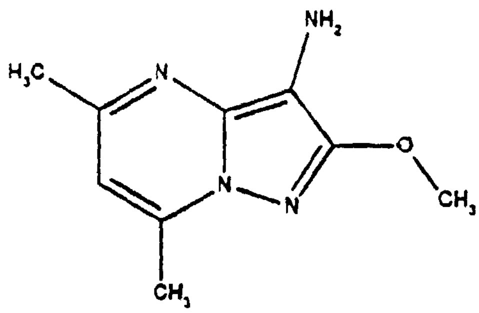

1,04 g 2-Methoxy-5,7-dimethyl-pyrazolo-[1,5-a]-pyrimidin (Ann. Chem. 647, 116 (1961)) werden analog Beispiel 3 nitrosiert. Man erhält 1,1 g (91 % der Theorie) der entsprechenden 3-Nitrosoverbindung die analog Beispiel 1.2 mit Zinkstaub reduziert wird. Man erhält 0,8 g (80 % der Theorie) der Titelverbindung mit Fp. 187-190 °C.
DC (Kieselgel, Aceton/Methylenchlorid/Eisessig 50:45:5): Rf = 0.6

Beispiel 6

Analog Beispiel 5 erhält man die in der nachfolgenden Tabelle aufgeführten Verbindungen durch Nitrosieren des 3-H-Ausgangsheterocylus und Reduktion der Nitrosogruppe.

Der Ausgangsheterocyclus von Beispiel 6 b) ist beschrieben in J. Het. Chem. 12, 481 (1975), von Beispielen 6 c) und 6 d) in J. Het. Chem. 18, 1149 (1981). Der 3-H-Ausgangsheterocyclus des Beispiels 6 a) wird analog der entsprechenden 2-Methoxyverbindung in Beispiel 1 durch Alkylierung mit Bromessigsäureethylester (analog Bull. Chem. Soc. Jap. 49, 1980 (1976) hergestellt.

Tabelle

| Beispiel Nr. | Struktur | Fp. | $R_f$ 1) |
|---|---|---|---|
| 6 a | 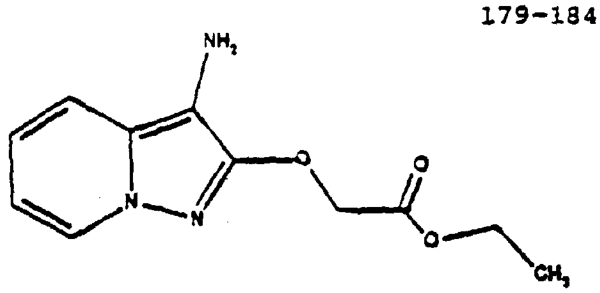 | 179-184 °C | 0,75 2) |
| 6 b | 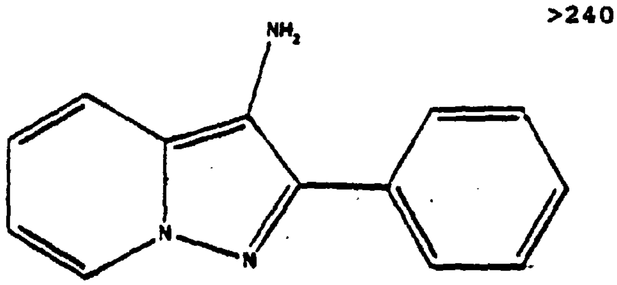 | >240 °C | 0,75 3) |
| 6 c | 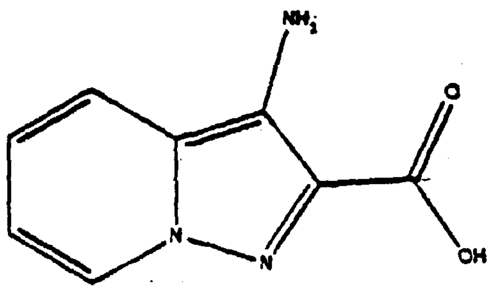 | >250 °C | 0,35 4) |
| 6 d | 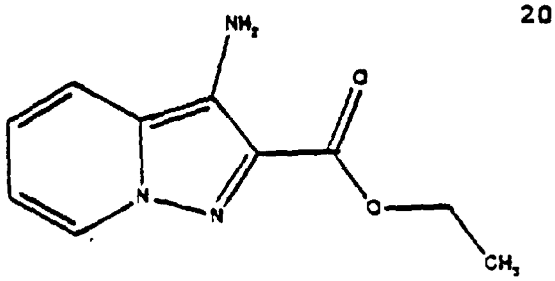 | 205-213 °C | 0,6 5) |

1) DC an Kieselgel

2) Laufmittel: Aceton/Methylenchlorid/Eisessig 50:45:5

3) Laufmittel: Methanol

4) Laufmittel: Essigsäureethylester/Aceton/Eisessig/ Wasser 50:25:12,5:12,5

5) Laufmittel: Essigsäureethylester

Beispiel 7

3-Amino-4-benzyl-pyrazolo-[1.5-a]-imidazol-dihydrochlorid

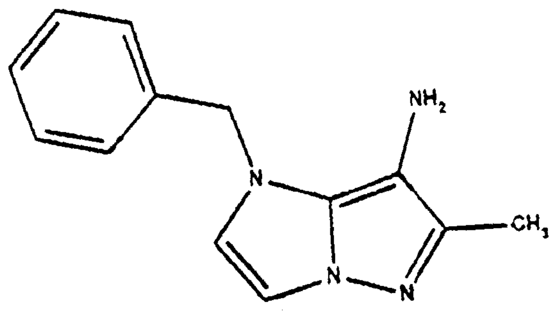

0,4 g 4-Benzyl-3-nitroso-pyrazolo-[1.5-a]-imidazol (Chem. Pharm. Bull. 22, 482 (1974)) werden analog Beispiel 1.2 mit Zink in Salzsäure reduziert und mit äthanolischer Chlorwasserstoffsäure in das Dihydrochlorid überführt. Man erhält 0,37 g (85 % der Theorie) 3-Amino-4-benzyl-pyrazolo-[1.5-a]-imidazol-dihydrochlorid mit Fp. 158 °C (Zersetzung).
DC (Kieselgel, Aceton/Methylenchlorid/Eisessig 50:45:5): Rf = 0,4

Beispiel 8

3-Amino-2-phenyl-pyrazolo-[1.5-a]-chinolin

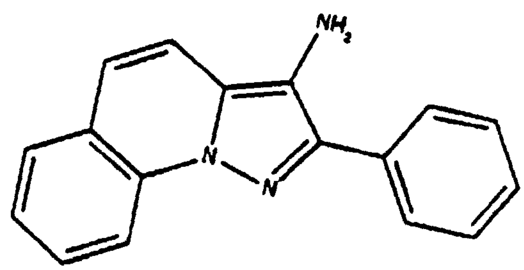

1.0 g 2-Phenyl-pyrazolo-[I.5-a] chinolin (J. Het. Chem. 12, 481 (1975)) werden analog Beispiel 3 nitrosiert. Man erhält 1 g (89 % der Theorie) der entsprechenden 3-Nitrosoverbindung, die in 90 ml Ethanol suspendiert und mit 200mg Palladium auf Kohle versetzt wird. Die Mischung wird auf 80 °C erhitzt und 0,25 ml Hydrazin-Hydrat werden portionsweise zugegeben. Nach 10 Minuten wird die Palladium-Kohle abfiltriert und das Filtrat eingedampft. Der Rückstand wird in Ethanol gelöst und mit äthanolischer Chlorwasserstoffsäure versetzt. Das ausgefallene Hydrochlorid wird abgesaugt. Man erhält 0,7 g (72 % der Theorie) der Titelverbindung mit Fp. 255-258 °C.
DC (Kieselgel, Methylenchlorid): Rf = 0,2

Beispiel 9

3-Amino-2,4-dimethyl-pyrazolo-[1.5-a]-imidazol-dihydrochlorid

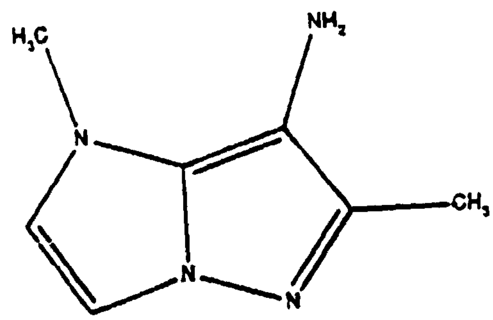

9.1 1,92 g 1,2-Dimethylimidazol werden in 10 ml Methylenchlorid gelöst und unter Eiskühlung mit einer Lösung von O-p-Toluolsulfonyl-hydroxylamin versetzt, die durch Umsetzung von 15,4 g O-p-Toluolsulfonyl-acethydroxamsäureethylester mit 118 ml 60 %iger wässriger Perchlorsäure erhalten wurde. Das Gemisch wird 3 Stunden bei Raumtemperatur gerührt. Der ausgefallene Niederschlag wird abfiltriert und mit Ether gewaschen. Man erhält 5 g (88 % der Theorie) N-Amino-1,2-dimethylimidazolium-p-toluolsulfonat.

9.2 5 g des in 9.1 erhaltenen Produktes werden mit 5.4 g Natriumacetat und 125 ml Acetanhydrid 1 Stunde auf 140 °C (Badtemperatur) erhitzt. Die Reaktionsmischung wird im Vakuum eingedampft und in Wasser aufgenommen. Man stellt den pH-Wert auf ca. 9-10 und extrahiert mit Methylenchlorid.
Die organische Phase wird getrocknet und eingedampft Der Rückstand wird an Kieselgel mit Essigsäureethylester chromatographiert. Man erhält 0.45 g (11 % der Theorie) 3-Acetyl-2,4-dimethyl-pyrazolo-[1.5-a]-imidazol mit Fp 165 -167 °C.

9.3 Die in 9.2 erhaltene Verbindung wird analog Beispiel 3 nitrosiert. Man erhält 0,35 g (94 % der Theorie) der entsprechenden 3-Nitrosoverbindung mit Fp. 179-183 °C, die in 100 ml verdünnter wässriger Natriumbicarbonatlösung gelöst wird und mit Natriumdithionit reduziert wird. Die Reaktionsmischung wird eingedampft und mit Ethanol digeriert. Die Ethanollösung wird eingeengt und das Produkt durch Zugabe von etherischer Chlorwasserstoffsäure ausgefällt. Man erhält 0,3 g (80 % der Theorie) der Titelverbindung mit Fp. 199-204 °C (Zers.).
DC (Kieselgel, Essigsäureethylester/Aceton/Eisessig/ Wasser = 50:25:12,5:12,5): Rf = 0,2

Beispiel 10

3-Amino-2,4-dimethyl-6-phenyl-pyrazolo-[3.2-c]-s-triazolhydrochlorid

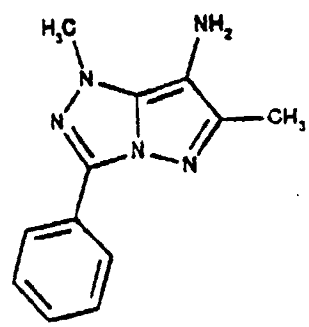

10.1 0,84 g 2-Methyl-6-phenyl-pyrazolo-[3.2-c]-s-triazol (J. Chem. Soc. Perkin I, 2047 (1977)) werden in 15 ml Dimethylformamid gelöst und mit 0,95 g p-Toluolsulfonsäuremethylester versetzt. Zu dieser Mischung gibt man portionsweise 0,2 g 55 %iges Natriumhydrid und rührt 1 Stunde bei Raumtemperatur. Das Reaktionsgemisch wird auf Eis gegossen und die Mischung mit Essigsäureethylester extrahiert. Man erhält 1 g rohes 2,4-Dimethyl-6-phenyl-pyrazolo-[3.2-c]-s-triazol. DC (Kieselgel, Methylenchlorid/Essigsäureethylester 50:1) Rf = 0,5.

10.2 Zu einer Lösung von 1,8 g 2,4-Dimethyl-6-phenyl-pyrazolo-[3.2-c]-s-triazol und 0,5 g Natriumacetat in 8 ml Eisessig gibt man portionsweise Phenyldiazoniumtetrafluoroborat, das durch Diazotierung von 2.2 ml Anilin erhalten wurde. Man läßt das Gemisch über Nacht stehen, verdünnt mit Wasser und saugt den orangegelben Niederschlag ab. Die 3-Phenylazoverbindung wird durch Chromatographie an Kieselgel mit Methylenchlorid gereinigt. Ausbeute 1,3 g (73 % der Theorie) mit Fp. 183-186 °C.

10.3 1,3 g 2,4-Dimethyl-6-phenyl-3-(phenyl-azo-pyrazolo-[3.2-c]-s-triazol aus 10.2 werden in einem Gemisch aus 100 ml Ethanol und 100 ml verdünnter wässriger Natriumbicarbonatlösung suspendiert und mit überschüssigem Natriumdithionit versetzt. Die Mischung wird ca. 2 Tage bei 60 °C gerührt. Während dieser Zeit gibt man noch Natriumdithionit, Natriumbicarbonat und Ethanol (1 ltr) zu. Die Reaktionsmischung wird eingedampft und der Rückstand in Essigsäureethylester aufgenommen. Die organische Phase wird gründlich mit Wasser gewaschen, getrocknet und eingedampft. Der Rückstand wird in Ethanol gelöst und durch Zugabe von ethanolischer Chlorwasserstoffsäure das Hydrochlorid ausgefällt. Man erhält 0,8 g der Titelverbindung mit Fp. 210-213 °C.
DC (Kieselgel, Essigsäureethylester/Aceton/Eisessig/ Wasser 50:25:12,5:12,5): Rf = 0,7

Beispiel 11

3-Amino-pyrazolo-[1.5-b]-pyridazin-hydrochlorid

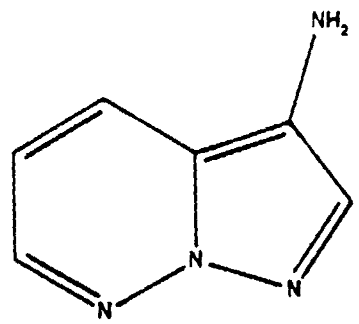

11.1 1,8 g Pyrazolo-[1.5-b]-pyridazin-3-carbonsäureethylester (Chem. Pharm. Bull. 22, 1814 (1974)) werden mit 36 ml 6N Salzsäure 5 Stunden unter Rückfluß gekocht. Die Reaktionsmischung wird eingedampft und der Rückstand in Wasser gelöst. Man neutralisiert mit Natriumbicarbonat und extrahiert 3 mal mit Essigsäureethylester. Die organische Phase wird getrocknet und eingedampft. Man erhält 0,85 g (75 % der Theorie) öliges Pyrazolo-[1.5-b]-pyridazin.
DC (Kieselgel, Methylenchlorid/Essigsäureethylester 2:1): Rf = 0,6.

11.2 0,8 g der in 11.1 erhaltenen Verbindung werden analog Beispiel 8 nitrosiert. Man erhält 0,6 g (60 % der Theorie) 3-Nitroso-pyrazolo-[1.5-b]-pyridazin mit Fp. 129-132 °C.
Analog Beispiel 8 wird die Nitroso-verbindung mit Palladium/Hydrazin reduziert. Man erhält 0,4 g (58 % der Theorie) der Titelverbindung mit Fp. 230 °C (Zers.).
DC (Kieselgel, Essigsäureethylester/Methanol 1:1): Rf = 0,6

Beispiel 12

3-Amino-2,6-dimethyl-pyrazolo-[1.5-a]-pyrimidin-hydrochlorid

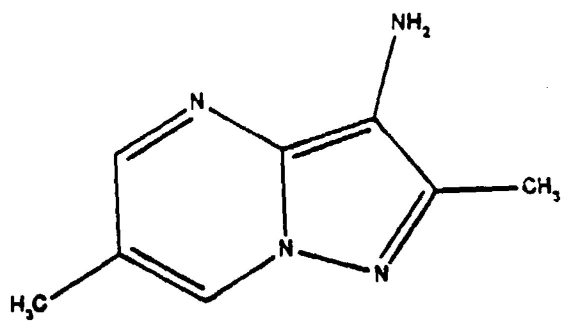

3,2 g 2,6-Dimethyl-3-nitro-pyrazolo-[1.5-a]-pyrimidin (Synthesis 673 (1982)) werden in 320 ml Ethanol gelöst und mit 320 ml 5 %iger Natriumbicarbonatlösung in Wasser versetzt. Zu diesem Gemisch gibt man unter Rühren und Kühlen portionsweise 14,2 g Natriumdithionit zu, bis das Dünnschichtchromatogramm die Abwesenheit von Ausgangsmaterial zeigt. Man engt das Reaktionsgemisch etwas ein und extrahiert 3 x mit Essigsäureethylester. Die organische Phase wird getrocknet und eingedampft. Der Rückstand wird in wenig Ethanol gelöst und mit einer äquimolaren Menge Chlorwasserstoff in Ether versetzt. Die ausgefallenen Kristalle werden abgesaugt. Man erhält 2,9 g (80 % der Theorie) der Titelverbindung mit Fp. 224 °C (Zersetzung). DC (Kieselgel, Chloroform/Methanol/Methylethylketon/Eisessig/Wasser (75:35:25:5:8): Rf = 0,73.

Beispiel 13

3-Amino-4-methyl-6-methylthio-2-phenyl-pyrazolo-[3,2-c]-s-triazol-hydrochlorid

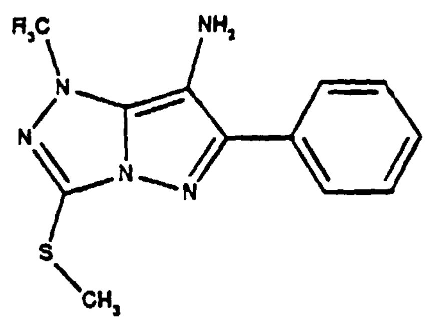

13.1 4,6 g 6-Methylthio-2-phenyl-pyrazolo(3.2-c)-s-triazol (J. Chem. Soc. Perkin I, 2047 (1977)) werden in 46 ml trockenem Dimethylformamid gelöst und mit 4,4 g p-Toluolsulfonsäure-methylester versetzt. Dazu gibt man portionsweise unter Rühren 1 g ca. 55 %iges Natriumhydrid. Die Mischung wird 1 Stunde bei Raumtemperatur gerührt und dann auf Eis/Wasser gegossen. Man extrahiert mit Essigsäureethylester, trocknet die organische Phase und dampft zur Trockene ein. Das Rohprodukt wird durch Chromatographie an Kieselgel mit Methylenchlorid/Essigsäureethylester (50:1) gereinigt. Man erhält 1,5 g (30 % der Theorie) des an N-4 methylierten Heterocyclus.
DC (Kieselgel, Methylenchlorid/Essigsäureethylester 50:1): Rf = 0,35.

13.2 1,2 g der in 13.1 erhaltenen Verbindung werden in 36 ml Eisessig gelöst und mit 1,33 g 4-Methoxyphenyldiazonium-tetrafluoroborat versetzt. Nach 2 Stunden gibt man nochmals die gleiche Menge des Diazoniumsalzes zu und rührt das Gemisch über Nacht. Das Reaktionsgemisch wird mit Wasser verdünnt und der pH mit verdünnter wässriger Natronlauge auf 5 gestellt. Der Niederschlag wird abfiltriert, mit Wasser gewaschen und bei 50 °C im Vakuum getrocknet. Man erhält 1.9 g (100 % der Theorie) der entsprechenden 3-Azoverbindung.

13.3 Die in 13.2 erhaltene Azoverbindung wird in 100 ml Eisessig suspendiert und durch Zugabe von Zinkstaub reduziert. Das überschüssige Zink wird abfiltriert und die Reaktionsmischung eingedampft. Zur Reinigung wird

das Rohprodukt in 100 ml Dioxan gelöst und mit 1,5 g Di-tert.-Butyldicarbonat versetzt. Man rührt das Gemisch 2 Stunden, dampft ein und chromatographiert das Produkt an Kieselgel mit Methylenchlorid/Essigsäureethylester (100:0 bis 90:10). Die Fraktionen, die die gewünschte N-tert.-butoxycarbonylierte Aminoverbindung enthält, werden vereinigt, eingedampft und in 50 ml Ethanol suspendiert. Man gibt zur Abspaltung der t-Butoxycarbonylgruppe 50 ml ethanolische Chlorwasserstoffsäure zu und rührt bei Raumtemperatur. Das Gemisch wird zur Trockene eingedampft, in Ethanol heiß gelöst, filtriert und das Filtrat mit Ether versetzt. Der Niederschlag wird abgesaugt. Man erhält 0,6 g (40 % der Theorie) der Titelverbindung mit Fp. 207-210 °C.
DC (Kieselgel, Essigsäureethylester): Rf = 0,65

Beispiel 14

3-Amino-4-chlor-2-methoxy-pyrazolo-[1.5-a]-pyridinhydrochlorid

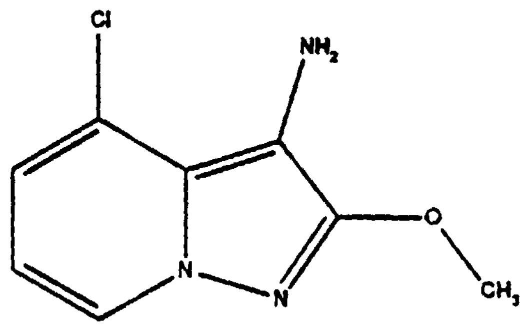

7,5 g N-Amino-3-chlorpyridinium-mesitylensulfonat (J. Chem. Soc. Perkin I, 2580 (1973)) werden in 50 ml trockenem Dimethylformamid gelöst und mit 3,5 g fein gepulvertem Kaliumcarbonat versetzt. Unter Kühlung gibt man 28 ml einer 5 m Diketenlösung in Methylenchlorid zu. Die Mischung wird 30 Minuten bei 0 °C und 2 Stunden bei Raumtemperatur gerührt. Der ausgefallene Niederschlag wird abgesaugt, das Filtrat eingedampft und der Rückstand durch Chromatographie an Kieselgel mit Methylenchlorid/5 -10 % Methanol gereinigt. Man erhält 2,7 g der N-acetoacetylierten Verbindung, die in 100 ml Ethanol gelöst und mit 2,6 g fein gepulvertem Kaliumcarbonat versetzt wird. Das Gemisch wird 2 Tage bei Raumtemperatur gerührt und schließlich eingedampft. Der Rückstand wird in 100 ml trockenen Dimethylformamid gelöst, die Lösung abgekühlt und mit 7 g Methyljodid versetzt. Man rührt das Gemisch 1 Stunde bei 0 °C und 30 Stunden bei Raumtemperatur. Das Dimethylformamid wird abdestilliert, der Rückstand in Wasser/Essigsäureethylester gelöst. Die Essigesterphase wird abgetrennt, getrocknet und eingedampft. Das Produkt wird durch Säulenchromatographie an Kieselgel mit Methylenchlorid/Essigsäureethylester 20:1 gereinigt.

Man erhält 0,8 g 3-Acetyl-4-Chlor-2-methoxy-pyrazolo-[1.5-a]-pyridin, das analog Beispiel 3 nitrosiert und reduziert wird.
Ausbeute: 0,3 g der Titelverbindung
Fp. 200-230 °C (Zers.)
DC (Kieselgel, Methylenchlorid/Essigsäureethylester 1:1): Rf = 0,6

Beispiel 15

3-Amino-2-methyl-5,6,7,8-tetrahydro-pyrazolo-(3.2-b)-benzothiazol-hydrochlorid

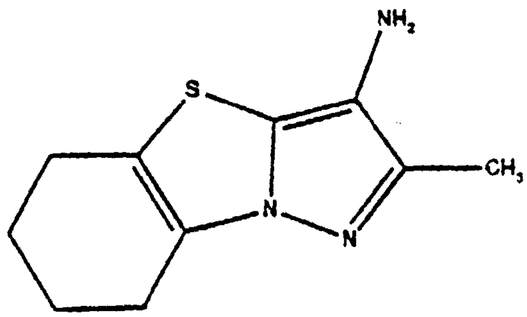

15.1 28,5 g Acetamid und 21,3 g Phosphorpentasulfid werden in 32 ml absolutem Toluol suspendiert und unter Rühren mit 5 ml eines Gemisches aus 85 g 2-Bromcyclohexanon und 40 ml absolutem Toluol versetzt. Man erhitzt auf 75-80 °C und gibt den Rest der Bromketonlösung zu. Das Gemisch wird 10 Minuten unter Rückfluß gekocht, abgekühlt und mit 2 N Natronlauge auf pH 8-9 gestellt. Man extrahiert mit Essigsäureethylester, wäscht die organische Phase mit Wasser, trocknet und dampft ein. Der Rückstand wird im Vakuum destilliert. Man erhält 14,4 g 2-Methyl-4,5,6,7-tetrahydrobenzothiazol

Kp. 62°C/ 66,66 Pa (0,5 mm Hg)

15.2 2,06 g 2-Methyl-4,5,6,7-tetrahydrobenzothiazol werden in ca. 10 ml Methylenchlorid gelöst, die Mischung abgekühlt und mit 30 mMol einer Methylenchloridlösung von O-p-Toluolsulfonylhydroxylamin versetzt. Man rührt 2 Stunden im Eisbad und fällt das Produkt durch Zugabe von Ether aus. Man erhält 6.4 g (94 % der Theorie) N-Amino-2-methyl-4,5,6,7-tetrahydrobenzothiazolium-p-toluolsulfonat mit Fp. 127-140 °C.

15.3 6,2 g der in 15.2 erhaltenen N-Aminoverbindung werden zusammen mit 4.7 g Natriumacetat und 16 ml Acetanhydrid auf 140 °C (Badtemperatur) erhitzt. Nach 1 Stunde wird das Gemisch eingedampft, der Rückstand in Wasser/Methylenchlorid gelöst, mit Soda alkalisch gestellt und dreimal mit Methylenchlorid extrahiert. Das Produkt wird durch Säulenchromatographie an Kieselgel mit Essigsäureethylester/Ligroin (1:2) gereinigt. Man erhält 2,1 g (50 % der Theorie) 3-Acetyl-2-methyl-5,6,7,8-tetrahydropyrazolo-[3.2-b]-benzothiazol mit Fp. 90-92 °C.

15.4 Die in 15.3 erhaltene Verbindung (1,4 g) wird analog Beispiel 3 nitrosiert und die Nitrosoverbindung reduziert. Man erhält 1,3 g der Titelverbindung mit Fp 239-240 °C (Zers.).
DC (Kieselgel, Essigsäureethylester): Rf = 0,5

Beispiel 16

3-Amino-2,5-dimethyl-6-phenyl-pyrazolo-[3.2-b]-thiazolhydrochlorid

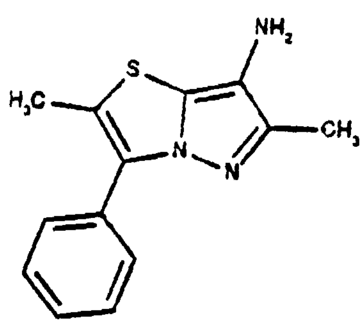

Analog Beispiel 15 erhält man, ausgehend von α-Brompropiophenon die Titelverbindung mit Fp. 252-254 °C (Zers.) DC (Kieselgel, Essigsäureethylester): Rf = 0.6

Beispiel 17

3-Amino-pyrazolo-[1.5-a]-pyridin-hydrochlorid

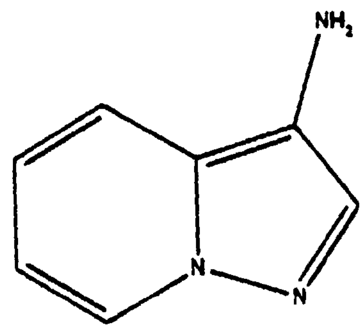

17.1 8,2 g Pyrazolo-[1.5-a]-pyridin (Ann. Chem. 498 (1977)) werden in 6 N Salzsäure (30 ml) gelöst, die Lösung auf 0 °C gekühlt und dazu langsam eine Lösung von 6.9 g Natriumnitrit in 30 ml Wasser getropft. Nach 1 Stunde ist die Nitrosierung beendet. Man setzt ca. 100 ml Wasser zu und extrahiert mehrfach mit Essigsäureethylester. Die organische Phase wird getrocknet und eingeengt. Man erhält 9,6 g 3-Nitroso-pyrazolo-[1.5-a]-pyridin.

17.2 9 g der oben erhaltenen Nitrosoverbindung werden in eine Lösung von 22 g Zinn(II)-chlorid-Dihydrat in 180 ml konzentrierter Salzsäure eingetragen. Die Reaktionsmischung wird 1 Stunde bei Raumtemperatur gerührt und zur Vervollständigung der Reduktion mit 8 g Zinn (II)-chlorid Dihydrat in 30 ml konzentrierter Salzsäure versetzt. Die Suspension wird auf ca. 150 g Eis gegossen, mit Natriumhydroxid auf pH 12 gestellt und rasch mit Essigsäureethylester extrahiert. Die Essigesterphase wird getrocknet und eingeengt. Der Rückstand wird in ca. 350 ml Ether gelöst und mit etherischer Chlorwasserstoffsäure versetzt. Der ausgefallene Niederschlag wird abfiltriert, mit Ether gewaschen und getrocknet. Man erhält 11,3 g (100 % der Theorie) der Titelverbindung mit Fp. 228-232 °C DC (Kieselgel, Essigsäureethylester/Methanol 9:1): Rf = 0,52

Beispiel 18

Herstellung von 3-Amino-2-methylthio-pyrazolo-[1,5-a]-pyridin-hydrochlorid

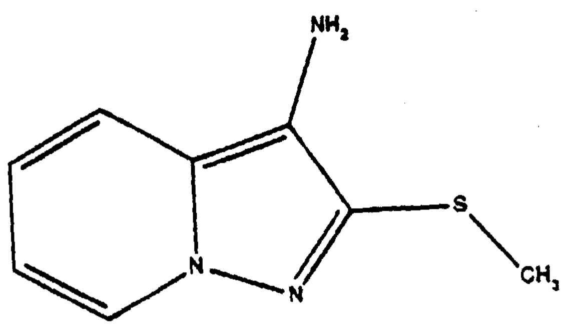

4 g 2-Methylthio-3-nitro-pyrazolo-[1,5-a]-pyridin (Heterocycles 6, 379 (1977), Chem. Pharm. Bull. 25, 1528 (1977) werden in 200 ml konzentrierter Salzsäure gelöst und mit 20 g Zinn(II)-chlorid-Dihydrat versetzt. Nach einer Stunde werden nochmals 15 g Zinn(II)chlorid-Dihydrat, 15 ml konzentrierte Salzsäure und 200 ml Wasser zugegeben. Nach einer weiteren Stunde Reaktionszeit wird die Mischung auf Eis gegossen, die gelbe Lösung mit Natriumhydroxid alkalisch gestellt und mit Essigsäureethylester extrahiert. Die organische Phase wird getrocknet und eingedampft. Der Rückstand wird in wenig Ethanol gelöst und mit etherischer Chlorwasserstoffsäure versetzt, um das Hydrochlorid zu bilden.

Man erhält 3,9 g (95 % der Theorie) der Titelverbindung mit Fp. 226 °C.
DC (Kieselgel, Methylenchlorid/tert. Butylmethylether 2:8): Rf = 0,6.

Beispiel 19

R,S-3-Amino-2-(1-hydroxyethyl)-pyrazolo-[1,5-a]-pyridin-hydrochlorid

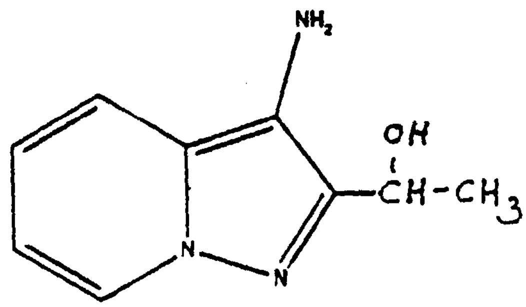

25 g N-Aminopyridin-hydrojodid werden in 250 ml trockenem Dimethylformamid gelöst und unter Rühren mit 17,5 g Kaliumcarbonat versetzt. Anschließend tropft man unter Rühren 20,5 g R,S-2-Hydroxy-5-oxo-butin-3 (J. Chem. Soc. Perkin I 1908 (1972)) zu. Die Mischung erwärmt sich dabei und wird über Nacht stehengelassen. Nach Zugabe von 1,25 1 Wasser wird mehrfach mit Essigsäureethylester extrahiert. Die vereinigten Extrakte werden getrocknet und eingedampft. Das verbleibende Öl wird mit etwas Ether verdünnt. Die nach einiger Zeit ausgefallenen Kristalle werden abgesaugt. Man erhält 9,8 g (42 % der Theorie) R,S-3-Acetyl-2-(1-hydroxyethyl)-pyrazolo-[1,5-a]-pyridin ($R_f$ (Kieselgel, Methylenchlorid/Essigsäureethylester 1:1) = 0,35), das analog Beispiel 3 nitrosiert wird. Die Nitrosoverbindung wird analog Beispiel 8 mit Palladium/Kohle/Hydrazinhydrat reduziert. Man erhält 6,8 g der Titelverbindung, deren Kristalle 1,8 Mol HCl enthalten und bei 229-231 °C (Zers.) schmelzen.
$R_f$ (Kieselgel, Essigsäureethylester/Methanol 3:1) = 0,65

Beispiel 20

3-Amino-2,5-dimethyl-7-(dimethylamino)-pyrazolo-[1,5-a]-pyrimidin-hydrochlorid

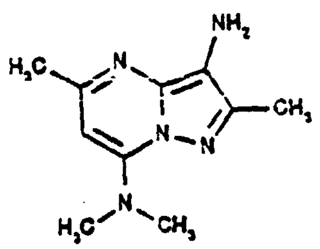

x HCl

20.1

1.6 g 2,5-Dimethyl-7-hydroxy-pyrazolo-[1,5-a]-pyrimidin werden mit 16.6 ml Phosphoroxychlorid und 0.8 ml N,N-Dimethylanilin 40 min unter Rückfluß erhitzt. Das überschüssige Phosphoroxychlorid wird abdestilliert und der Rückstand auf Eis gegossen. Man extrahiert mit Methylenchlorid, wäscht die organische Phase mit $Na_2CO_3$-Lösung, trocknet und engt ein. Der Rückstand wird in 28 ml Ethanol gelöst und mit 2 g einer 40proz. Lösung von Dimethylamin in Wasser versetzt. Die Mischung wird 2,5 Stunden bei Raumtemperatur gerührt, eingedampft und der Rückstand über Kieselgel mit Essigsäureethylester chromatographiert. Man erhält 0,86 g (46 % der Theorie) 2,5-Dimethyl-7-(N,N-dimethylamino)-pyrazolo-[1,5 a]-pyrimidin das analog Beispiel 3 nitrosiert wird. Die Nitrosoverbindung wird analog Beispiel 8 mit Palladium/Kohle/Hydrazinhydrat reduziert. Man erhält 0,8 g der Titelverbindung ($R_f$ 0,54, Kieselgel, Chloroform/Methanol/ Methylethylketon/Eisessig/Wasser= 75:35:25:5:8).

Beispiel 21

3-Amino-4-methyl-pyrazolo-[1,5-a]-imidazol-hydrochlorid

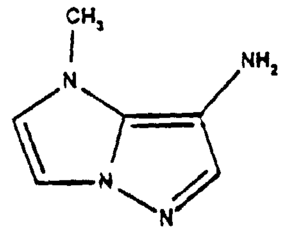

x HCl

21.1

Analog dem in "Organikum" (VEB Deutscher Verlag der Wissenschaften, Berlin) auf S. 514/515 beschriebenen Verfahren setzt man 65 g Ethoxymethylencyanessigsäureethylester mit 55 g 2,2-Diethoxyethylhydrazin um und erhält 100,2 g 5-Amino-1-(2,2-diethoxyethyl)-pyrazol-4-carbonsäure, die in 4 1 Ethanol gelöst und mit 2 1 20proz. Schwefelsäure versetzt wird.

Man erhitzt 3 Stunden unter Rückfluß und neutralisiert durch Zugabe von festem Natriumbicarbonat. Von ausgefallenem Salz wird abfiltriert und das Filtrat eingeengt. Der Rückstand wird mehrfach mit siedendem Methylenchlorid ausgezogen. Die Filtrate werden vereinigt. Man erhält 58,2 g (86 % der Theorie) Pyrazolo-[1.5-a]-imidazol-3-carbonsäureethylester, vom Fp. 126-127° C. ($R_f$ = 0,64, Essigsäureethylester, Kieselgel)

21.2

18,7 g des oben erhaltenen Carbonsäureesters werden in 190 ml Dimethylformamid gelöst und mit 17 g p-Toluolsulfonsäuremethylester versetzt. Unter Rühren werden 3,98 g Natriumhydrid (55proz.) portionsweise eingetragen. Man rührt 30 Minuten bei Raumtemperatur, gießt die Mischung auf Eis/Wasser und extrahiert mit Essigsäureethylester. Man erhält 21,9 g (100 %) des 4-Methylpyrazolo-[1,5-a]-imidazol-3-carbonsäureethylesters, der durch Kochen in 600 ml konzentrierter Salzsäure verseift wird. Gleichzeitig decarboxyliert die entstehende Carbonsäure und man erhält 16,7 g (83 % der Theorie) 4-Methylpyrazolo-[1,5-a]-imidazol-dihydrochlorid ($R_f$ = 0,39, Kieselgel, Methylenchlorid mit 5 Vol.-% Methanol).

21.3

12.8 g Anilin werden in einer Lösung von 12,8 ml konzentrierter Schwefelsäure in 64 ml Wasser durch Zugabe einer Lösung von 9,5 g Natriumnitrit in 42 ml Wasser diazotiert. Die Lösung wird durch Zugabe von NaOH auf pH 5 gestellt und bei 5 - 10° C tropfenweise mit einer Lösung von 16,5 g des oben erhaltenen 4-Methyl-pyrazolo-[1.5-a]-imidazol-dihydrochlorids in 165 ml Wasser und 14 ml Eisessig versetzt. Man rührt 1 Stunde bei 5 - 10° C und 3 Stunden bei Raumtemperatur und saugt dann den ausgefallenen Niederschlag ab. Man erhält 12 g 4-Methyl-3-phenylazo-pyrazolo-[1.5-a]-imidazol das analog Beispiel 10.3 mit Natriumdithionit reduziert und analog Beispiel 13.3 gereinigt wird. Man erhält 3,3 g 3-Amino-4-methyl-pyrazolo-[1.5-a]-imidazol-hydrochlorid. Fp. 210° C (Zers.) $R_f$ = 0,23, Kieselgel, Essigsäureethylester/Aceton/Eisessig/Wasser= 50:25:12,5:12,5).

Beispiel 22

3-Amino-2,4,6-trimethyl-pyrazolo-[3,2-c]-s-triazol-hydrochlorid

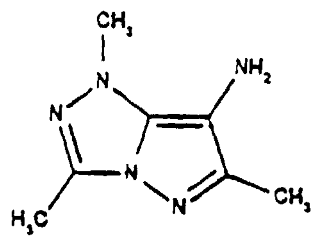

x HCl

22.1

Ausgehend von 4-Ethoxycarbonyl-3-methyl-pyrazol-5-ylhydrazin (Chem. Ber. 89, 2552 (1956) stellt man wie in DE-A-1810462 (Beisp. 6) beschrieben 2,6-Dimethyl-4H-pyrazolo-[3,2-c]-s-triazol-3-carbonsäureethylester her, der vor der sauren Verseifung analog Beispiel 10.1 methyliert wird. Als Zwischenprodukt erhält man so 2,4,6-Trimethyl-pyrazolo-[3,2-c]-s-triazol-3-carbonsäureethylester ($R_f$ = 0,52; Kieselgel, Essigsäureethylester/Ligroin=1:1), der durch Kochen mit konzentrierter Salzsäure verseift und gleichzeitig decarboxyliert wird. Man erhält 2,4,6-Trimethyl-pyrazolo-[3,2-c]-s-triazol ($R_f$ = 0.24, Kieselgel, Essigsäureethylester/Ligroin=1:1) als schwach gelbliches Öl, das nach einiger Zeit kristallisiert.

22.2

Analog Beispiel 13.2 und 13.3 wird das oben erhaltene Produkt mit p-Methoxybenzoldiazoniumsalz umgesetzt, reduziert und das Produkt gereinigt. Man erhält die Titelverbindung. Fp. 240° C ($R_f$ = 0,50, Kieselgel, Essigsäureethylester/Aceton/Eisessig/Wasser = 50:25:12,5:12,5).

Beispiel 23

2-Acetamido-3-amino-pyrazolo-[1,5-a]-pyridin-hydrochlorid

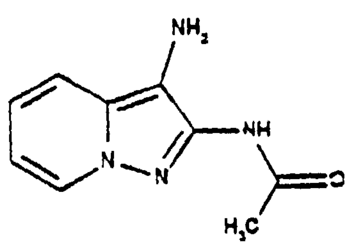

x HCl

4 g 2-Acetamido-pyrazolo-[1,5-a]-pyridin (Chem. Pharm. Bull. 21, 2146 (1973)) werden analog Beispiel 3 nitrosiert. Die Nitrosoverbindung wird analog Beispiel 8 mit Palldium/Kohle/Hydrazinhydrat reduziert. Man erhält 3,9 g (67 % der Theorie) 2-Acetamido-3-amino-pyrazolo-[1,5-a]-pyridin-hydrochlorid vom Fp. 255-259°C (Zersetzung).

Rf = 0,5; Kieselgel, Essigsäureethylester/Aceton/Eisessig/ Wasser = 50:25:12,5:12,5

Beispiel 24

2-Vinyl-3-amino-pyrazolo-[1,5-a]-pyridin-hydrochlorid

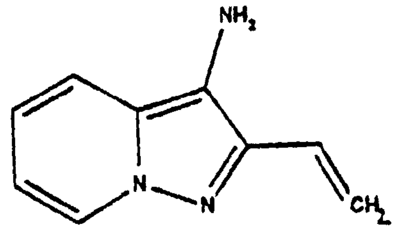

x HCl

25 g R,S-3-Acetyl-2-(1-hydroxyethyl)-pyrazolo-[1.5-a]-pyridin (aus Beispiel 22) werden mit 50 ml konzentrierter Schwefelsäure versetzt und bei 95° C Badtemperatur 2 Stunden erhitzt. Die Mischung wird auf viel Eis gegossen, mit NaOH alkalisch gestellt und mit Essigsäureethylester extrahiert. Das Rohprodukt wird über Kieselgel mit Ligroin/Essigsäuerethylester (95:5 bis 90:10) chromatographiert. Man erhält 3,4 g 2-Vinyl-pyrazolo-[1,5-a]-pyridin ($R_f$ = 0,6; Kieselqel, Ligroin/Essigsäureethylester=3:1), das analog Beispiel 21.2 mit Phenyldiazoniumsalz umgesetzt, reduziert und gereinigt wird. Man erhält die Titelverbindung.
$R_f$ = 0,65; Kieselgel, Ligroin/Aceton/Eisessig=50:45:5)

Beispiel 25

6-(3-Acetoxypropyl)-3-amino-2-methyl-pyrazolo-[1,5-a]pyrimidin

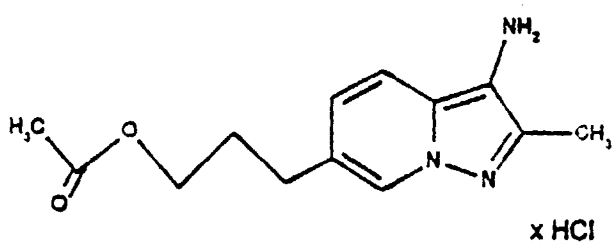

x HCl

Analog Beispiel 12 erhält man durch Reduktion von 6-(3-Acetoxypropyl)-2-methyl-3-nitro-pyrazolo-[1,5-a]-pyrimidin (Synthesis S. 673 (1982)) die Titelverbindung.
$R_f$ = 0,21 (Kieselgel, Methylenchlorid/Methanol=90:1)

Beispiel 26

2,3-Diamino-pyrazolo-[1,5-a]-pyridin-dihydrochlorid

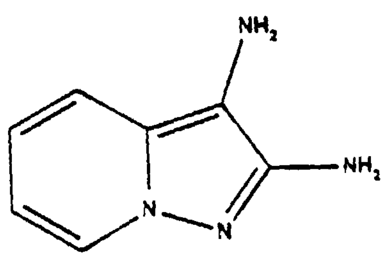

x HCl

2,66 g 2-Amino-pyrazolo-[1,5-a]-pyridin (Chem. Pharm. Bull. 21, 2146 (1973) werden analog Beispiel 13.2 mit p-Methoxy-benzoldiazoniumsalz umgesetzt.

Die entstandene Azoverbindung wird analog Beispiel 10.3 mit Natriumdithionit reduziert und das Rohprodukt analog Beispiel 13.3 gereinigt. Man erhält die Titelverbindung vom Fp. 190° C ($R_f$ = 0,6; Kieselgel, Ligroin/Aceton/Eisessig-60:40:1).

Beispiel 27

3-Amino-pyrazolo-[3,2-b]-thiazol-hydrochlorid

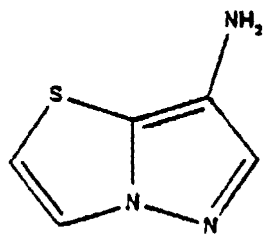

x HCl

27.1

60 g des Kaliumsalzes der Dithiocarbazinsäure werden in 400 ml Dimethylformamid bei 50° C Badtemperatur 12 Stunden mit 60 ml Bromacetaldehyddiethylacetal umgesetzt. Das Gemisch wird eingedampft, die verbleidende Emulsion mit 500 ml Wasser versetzt und das Produkt mit Ether extrahiert. Das Rohprodukt wird durch Chromatographie über Kieselgel (Ether/Ligroin=6:4) gereinigt. Man erhält 20,9 g des Dithiocarbazinsäure-(2,2-diethoxyethyl)esters.

27.2

17,75 g des oben erhaltenen Carbazinsäureesters und 11,3 g Formyl-chloressigester werden in 250 ml Ethanol gelöst und 2 Stunden auf 50° C erhitzt. Die Mischung wird eingeengt, mit Wasser versetzt und das Produkt mit Ether extrahiert. Die Etherphase wird getrocknet und eingeengt. Der Rückstand wird in 300 ml ethanolischer Salzsäure 4 Stunden unter Rückfluß erhitzt. Das Reaktionsgemisch wird eingedampft, der Rückstand in Wasser aufgenommen und das Reaktionsprodukt mit Essigsäureethylester extrahiert. Zur Reinigung wird über Kieselgel mit Essigsäureethylester/ Ligroin=1:1 chromatographiert. Man erhält 6,5 g Pyrazolo-[3,2-c]-thiazol-3-carbonsäureethylester, $R_f$ = 0,65; Toluol/Dioxan/Eisessig= 72:20:8

27.3

5,8 g des oben erhaltenen Esters werden in einem Gemisch aus 300 ml 3 N Salzsäure und 15 ml Dioxan 7 Stunden unter Rückfluß erhitzt, wobei alle zwei Stunden jeweils 15 ml konzentrierter Salzsäure zugegeben werden. Die Reaktionsmischung wird durch Zugabe von NaOH auf pH 8 gestellt und mehrfach mit Essigsäureethylester extrahiert. Nach dem Trocknen und Einengen der organischen Phase erhält man 3 g Pyrazolo-[3,2-c]-thiazol in Form eines gelblichen Öls. Dieses Produkt wird analog Beispiel 3 nitrosiert und reduziert. Man erhält 3,4 g der Titelverbindung als Dihydrochlorid vom Fp. 170° C (Zersetzung), $R_f$ = 0,66; Kieselgel, Essigsäureethylester/Aceton/Eisessig/Wasser = 50:25:12,5:12,5

Beispiel 28

3-Amino-4,6-dimethyl-pyrazolo-[3,2-c]-s-triazol-hydrochlorid

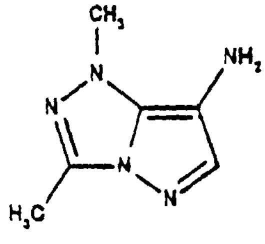

x HCl

28.1

24,4g 3-Amino-pyrazol-4-carbonsäureethylester (Organikum, S. 514, VEB Verlag der Wissenschaften, Berlin) werden in 100 ml konzentrierter Salzsäure suspendiert und bei 0 - 5° C durch Zugabe einer Lösung von 10,35 g Natriumnitrit in 50 ml Wasser diazotiert. Danach gibt man eine Lösung von 100 g Zinn(II)-chlorid in 100 ml konzentrierter Salzsäure zu und rührt die Mischung noch 2 Stunden im Eisbad. Der gelbe Niederschlag wird abfiltriert, in 150 ml Wasser gelöst und mit 20 ml Acetanhydrid versetzt. Die Mischung wird auf 80° C Badtemperatur erhitzt und portionsweise während 2 Stunden mit insgesamt 25 g Natriumbicarbonat versetzt.

Die neutrale Lösung wird dann 24 Stunden kontinuierlich mit Essigsäureethylester extrahiert. Nach dem Trocknen und Einengen der organischen Phase erhält man 17 g 3-Acethydrazinopyrazol-4-carbonsäureethylester: $R_f$ = 0,33, Kieselgel, Aceton/ Methylenchlorid/Eisessig=50:45:5), der mit Phosphoroxychlorid analog dem in DE-A 1810462 (Beispiel 6) beschriebenen Verfahren cyclisiert wird. Man erhält 4,8 g (32 % der Theorie) 6-Methyl-pyrazolo-[3,2-c]-s-triazol-3-carbonsäureethylester ($R_f$ = 0,70; Kieselgel, Toluol/Essigsäureethylester/Methanol=2:1:1).

28.2

Analog Beispiel 10.1 wird der oben erhaltene Heterocyclus methyliert und das Produkt durch Chromatographie über Kieselgel mit Essigsäureethylester/Ligroin=2:1 gereinigt. Durch 6stündiges Erhitzen mit 6 N Salzsäure wird der Ester verseift und die als Zwischenprodukt entstehende Carbonsäure decarboxyliert. Die Säure wird durch Zugabe von Natriumcarbonat neutralisiert und das Produkt mit Essigsäureethylester extrahiert. Man erhält 1,1 g öliges 4,6 Dimethyl-pyrazolo-[3,2-c]-s-triazol ($R_f$ = 0,26; Kieselgel, Essigsäureethylester/Ligroin=1:1).

28.3

Der oben erhaltene Heterocyclus wird analog Beispiel 3 nitrosiert und die Nitrosogruppe analog Beispiel 8 mit Palladium/Kohle/Hydrazin reduziert. Man erhält die Titelverbindung als Hydrochlorid vom Fp. 190°C (Zersetzung) $R_f$ = 0,53; Kieselgel, Essigsäureethylester/Aceton/Eisessig/ Wasser= 50:25:12,5:12,5

Beispiel 29

3-Amino-2-hydroxymethyl-pyrazolo-[1,5-a]-pyridin-hydrochlorid

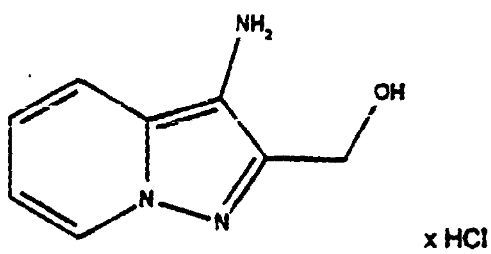

29.1

10,5 g Pyrazolo-[1,5-a]-pyridin-2-carbonsäureethylester (J. Het. Chem. 18, 1149 (1981)) werden in Tetrahydrofuran gelöst und zu einer Suspension von 5 g Natriumaluminiumhydrid in trockenem Tetrahydrofuran gegeben. Die Mischung wird 3 Stunden unter Rückfluß erhitzt, dann in eine Mischung aus Eis und Methanol gegossen. Man versetzt das Gemisch mit 100 ml 10prozentiger Ammoniumchloridlösung und extrahiert mit Essigsäureethylester. Die organische Phase wird getrocknet und eingeengt. Der Rückstand wird durch Chromatographie an Kieselgel mit Ligroin/Essigsäureethylester gereinigt.

Man erhält 7.3 g 2-Hydroxymethyl-pyrazolo-[1,5-a]-pyridin als gelbes Öl ($R_f$ = 0,49, Kieselqel, Essigsäureethylester)

29.2

Die oben erhaltene Substanz wird analog Beispiel 3 nitrosiert und analog Beispiel 8 mit Palladium/Kohle/Hydrazinhydrat reduziert. Man erhält die Titelverbindung vom Fp. 218°C. $R_f$ = 0,62; Kieselgel, Essigsäureethylester/Methanol =2:1

Beispiel 30

3-Amino-2-chlor-pyrazolo-[1,5-a]-pyridin-hydrochlorid

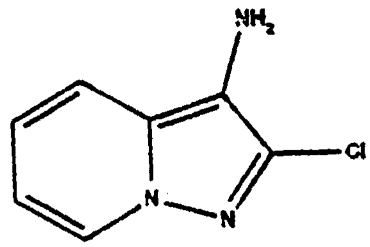

x HCl

2-Amino-pyrazolo-[1,5-a]-pyridin (Chem. Pharm. Bull. 21, 2146 (1973) wird analog Beispiel 13.2 mit p-Methoxybenzoldiazoniumsalz ungesetzt. 0,53 g der erhaltenen 3-Azoverbindung werden in 10 ml 6 N Salzsäure suspendiert und bei +5° C unter Rühren mit einer Lösung von 104 mg Natriumnitrit in 0,2 ml Wasser versetzt. Nach 30 Minuten gibt man eine kalte Lösung von 198 mg Kupfer(I)-chlorid in 6 ml 6 N Salzsäure zu und rührt die Mischung 40 Stunden bei Raumtemperatur. Die Mischung wird mit Wasser versetzt und 3 bis 4 mal mit Essigsäureethylester extrahiert. Man erhält nach der Reinigung des Rohproduktes durch Chromatographie über Kieselgel (Laufmittel: Essigsäureethylester Ligroin 1:1) 320 mg des 2-Chlor-3-(p-methoxyphenylazo)-pyrazolo-[1,5-a]-pyridins ($R_f$=0,65; Kieselgel, Essigsäure ethylester/Ligroin=1:1), das analog Beispiel 10.3 mit Natriumdithionit reduziert wird. Man erhält die Titelverbin dung vom Fp. 249 - 251° C (Zersetzung) $R_f$ = 0,2; Kieselgel, Essigsäureethylester/Ligroin=1:1

Beispiel 31

Analog Beispiel 17 erhält man ausgehend von substituierten Pyridinen die in der nachfolgenden Tabelle angegebenen Verbindungen.

| Ausgangsprodukt | Produkt | Fp. | $R_f$ |
|---|---|---|---|
| 31.1 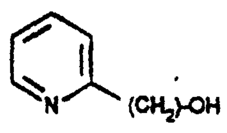 | 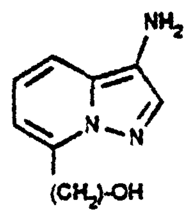 x HCl | 208 °C | 0.18 1) |
| 31.2 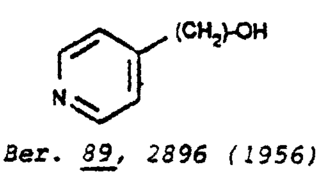 Chem.Ber. 89, 2896 (1956) | 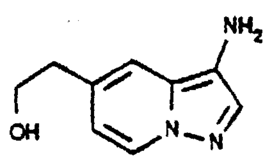 x HCl | 212 °C | 0.23 1) |

1) Essigester/ Methanol 9:1

Beispiel 32

3-Amino-2-morpholino-pyrazolo-[1,5-a]-pyridin-hydrochlorid

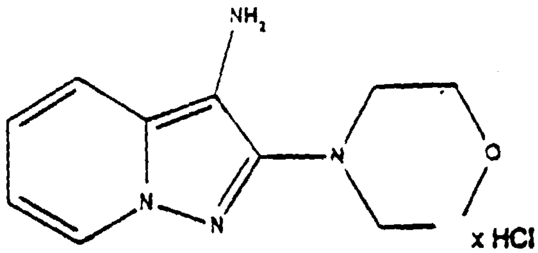

2-Amino-pyrazolo-[1,5-a]-pyridin (Chem. Pharm. Bull. 21, 2146 (1973) wird analog Beispiel 13.2 mit p-Methoxybenzoldiazoniumsalz umgesetzt. 4 g der Azoverbindung werden in 100 ml Dimethylformamid gelöst und mit 5,33 g β, β'-Dibromdiethylether und 2 g Natriumhydrid (55proz.) versetzt. Man rührt das Gemisch 1,5 Stunden bei Raumtemperatur und setzt Wasser und Essigsäureethylester zu. Die organische Phase wird abgetrennt und die wässrige Phase nochmals mit Essigsäureethylester extrahiert. Die vereinigten organischen Phasen werden getrocknet und eingeengt. Der Rückstand wird mit Hexan verrieben. Man erhält 4,75 g 3-(4-Methoxyphenyl)-azo-2-morpholino-pyrazolo-[1,5-a]-pyridin ($R_f$ = 0,7, Kieselgel, Essigsäureethylester/Methylenchlorid=1:1).

Die Azoverbindung wird analog Beispiel 13.3 mit Zink in Eisessig reduziert, gereinigt und die t-Butoxycarbonylgruppe mit HCl im Ethanol abgespalten. Man erhält 2,43 g der Titelverbindung vom Fp. 105 - 110°C (Zersetzung), $R_f$ = 0,4 (Kieselgel, Essigsäureethylester)

Beispiel 33

Bestimmung von Wasserstoffperoxid in wässriger Lösung.

Reagenzlösungen:

Lösung A

12 mg Peroxidase (= 3000U)
1 ml 0,1 n Phosphatpuffer, pH 8

Lösung B

53.6 mg 2,4,6-Tribromhydroxybenzoesäure
1 ml 0,1 n Phosphatpuffer, pH 8

Lösung C

32,1 mg N-Methyl-N-phenyl-amino-methanphosphonsäurehydrochlorid (hergestellt gemäß EP-A-0 175 250)
1 ml 0,1 n Phosphatpuffer, pH 8

4,8 x $10^{-7}$ Mol eines erfindungsgemäß anwendbaren Pyrazoloderivates werden gegebenenfalls unter Zuhilfenahme von wenig Methanol, Aceton oder Dimethylformamid in ca. 5-9 ml 0,1 n Phosphatpuffer pH 8 gelöst. Dazu gibt man 50 µl der Lösung A und je nach dem gewünschten Kuppler entweder 200 µl der Lösung B oder C. Schließlich fügt man noch 20 µl einer 0,012 molaren Lösung von $H_2O_2$ in Wasser zu, füllt mit dem Puffer auf 10 ml auf und mischt die Lösungen gut durch. Die Lösungen werden in 1 cm Küvetten vermessen. In der folgenden Tabelle 1 werden die Wellenlängen und Extinktionen bei λ max der entstandenen Farbstoffe aufgeführt.

Tabelle 1

| Substanz aus Beispiel Nr. | Kupplungskomponente TBHB 1) | | Kupplungskomponente Anilin 2) | |
|---|---|---|---|---|
| | λ max [nm] | Ext 3) | λ max [nm] | Ext. 3) |
| 1 | 568 | 69300 | 620 | 78200 |
| 2 | 536 | 18000 | 578 | 12600 |
| 3 | 622 | 28900 | 657 | 34800 |
| 4 | 572 | 30400 | 613 | 35600 |
| 5 | 557 | 36300 | 587 | 41900 |
| 6.1 | 568 | 39300 | 615 | 48800 |
| 6.2 | 628 | 19400 | 658 | 23000 |
| 6.3 | 602 | 22200 | 634 | 32600 |
| 6.4 | 587 | 7600 | 628 | 13300 |
| 7 | 626 | 30100 | 674 | 113300 |
| 8 | 613 | 13700 | 638 | 24200 |
| 9 | 617 | 45200 | 668 | 55500 |
| 10 | 613 | 39600 | 652 | 53300 |
| 11 | 504 | 10200 | 539 | 12600 |
| 12 | 542 | 39600 | 596 | 42000 |
| 13 | 612 | 25000 | 657 | 32600 |
| 14 | 577 | 42400 | 623 | 39300 |
| 15 | 594 | 34400 | 638 | 34000 |
| 16 | 592 | 34200 | 638 | 27400 |
| 17 | 572 | 53100 | 606 | 74600 |
| 18 | 635 | 31600 | 675 | 84600 |
| 19 | 613 | 22670 | 650 | 18160 |
| 20 | | | 639 | |
| 21 | 588 | 76290 | 633 | 94150 |
| 22 | 607 | 9300 | 648 | 42350 |
| 23 | 638 | 61500 | 658 | 66900 |
| 24 | 629 | 17950 | 660 | 28850 |

1) TBHB = 2,4,6-Tribrom-3-hydroxybenzoesäure
2) Anilin = N-Methyl-N-phenyl-aminomethanphosphonsäure Hydrochlorid
3) Ext = Extinktion in [Liter/Mol $cm^2$]

Tabelle 1 (Fortsetzung)

| Substanz aus Beispiel Nr. | Kupplungskomponente TBHB [1] | | Kupplungskomponente Anilin [2] | |
|---|---|---|---|---|
| | λ max [nm] | Ext [3] | λ max [nm] | Ext. [3] |
| 26 | 631 | 34620 | 683 | 35220 |
| 27 | 552 | 28940 | 585 | 39450 |
| 28 | 564 | 24210 | 604 | 41760 |
| 29 | 613 | 24870 | 648 | 37780 |
| 30 | 593 | 13990 | 631 | 22850 |
| 31.1 | 590 | 40970 | 623 | 51170 |
| 31.2 | 587 | 36950 | 619 | 46620 |
| 32 | 656 | 48450 | 709 | 134420 |

1) TBHB = 2,4,6-Tribrom-3-hydroxybenzoesäure
2) Anilin = N-Methyl-N-phenyl-aminomethanphosphonsäure Hydrochlorid
3) Ext = Extinktion in [Liter/Mol $cm^2$]

Beispiel 34

Bestimmung von Creatinin:

a) Indikatorsystem 2,4-6-Tribrom-3-hydroxybenzoesäure/Substanz aus Beispiel 4

Reagenz I:

| | |
|---|---|
| 100 mM | Tris-Puffer, pH 7,9 |
| 200 mM | Kaliumchlorid |
| 0.25% | Detergenz (Triton X 100®) |
| 5 mM | Natriumcholat |
| 10 mM | Ammoniumchlorid |
| 5 mM | Magnesiumchlorid |
| 15 mM | 2,4,6-Tribrom-3-hydroxybenzoesäure |
| 15 U/ml | Creatinin-imino-hydrolase (E.C. 3.5.4.21) |
| 0.5U/ml | N-Methylhydantoinase (DE-A-34 06 770) |
| 3 U/ml | Sarcosin-Oxidase (E.C. 1.5.3.1) |
| 3 U/ml | Peroxidase (E.C. 1.11.1.7) |

Reagenz II:

| | |
|---|---|
| 20 mM | Kaliumphosphat-Puffer, pH 6,0 |
| 100 mM | ATP |
| 3 mM | Substanz aus Beispiel 4 |
| 40 U/ml | N-Carbamoylsarcosin-Hydrolase (DE-A-32 48 145) |

Probematerial:

Wässrige Creatinin-Standardlösungen (2-20 mg/dl Creatinin)

Testansatz und Durchführung:

| | |
|---|---|
| Wellenlänge: | 569nm |
| Schichtdicke: | 10 mm |
| Temperatur: | 25 °C |
| Inkubationszeit: | 10 min |
| Pippetierschema: | |

| | |
|---|---|
| Reagenz I: | 1.00 ml |
| Reagenz II: | 50 µl |
| Probe: | 20 µl |

Messung gegen Reagenzienleerwert (Wasser statt Standardlösung als Probe)

Ergebnis:

| Standardkonzentration ( mg/dl ) | Absorption ( mE ) |
|---|---|
| 2 | 92 |
| 4 | 194 |
| 6 | 272 |
| 8 | 368 |

(fortgesetzt)

| Standardkonzentration ( mg/dl ) | Absorption ( mE ) |
|---|---|
| 10 | 472 |
| 12 | 565 |
| 14 | 658 |
| 16 | 753 |
| 18 | 841 |
| 20 | 974 |

b) Indikatorsystem N-Methyl-N-phenyl-aminomethanphosphonsäure (MPA)/Substanz aus Beispiel 4

Reagenz I:

Zusammenstellung wie bei Reagenz I in a), jedoch 2mM MPA (hergestellt gemäß EP-A-0 175 250) statt 15 mM 2,4,6-Tribrom-3-hydroxybenzoesäure

Reagenz II:

Zusammenstellung identisch mit Reagenz II in a)

Probematerial:

Wässrige Standardlösungen (2-20 mg/dl Creatinin)

Testansatz und Durchführung:

Wellenlänge: 620 nm
Schichtdicke, Temperatur, Inkubationszeit, Pipettierschema und Messung wie in a).

Ergebnis:

| Standardkonzentration ( mg/dl ) | Absorption ( mE ) |
|---|---|
| 2 | 61 |
| 4 | 125 |
| 6 | 184 |
| 8 | 249 |
| 10 | 307 |
| 12 | 369 |
| 14 | 429 |
| 16 | 491 |
| 18 | 554 |
| 20 | 615 |

c) Indikatorsystem N-Ethyl-N-3-sulfo-2-hydroxypropyl-m-anisidin (ADOS)/Substanz aus Beispiel 4

Reagenz I: Zusammensetzung wie bei Reagenz I in a), jedoch 2 mM ADOS (Chem. Pharm. Bull. 30, 2492 (1982)) statt 15 mM, 2,4,6-Tribrom-3-hydroxy-benzoesäure

Reagenz II: Zusammensetzung identisch mit Reagenz II in a)

Probematerial:

Wässrige Standardlösungen (2-20 mg/dl Creatinin)

Testansatz und Durchführung:

Wellenlänge: 598 nm
Schichtdicke, Temperatur, Inkubationszeit, Pippetierschema und Messung wie in a).

Ergebnis:

| Standardkonzentration ( mg/dl ) | Absorption ( mE ) |
|---|---|
| 2 | 48 |
| 4 | 107 |
| 6 | 165 |
| 8 | 227 |
| 10 | 280 |
| 12 | 338 |
| 14 | 386 |
| 16 | 450 |
| 18 | 500 |
| 20 | 565 |

Beispiel 35

Bestimmung von Glucose in Serum oder Vollblut mittels eines Testträgers

Die zum Nachweis erforderlichen Reagenzien werden in eine Filmbeschichtungsmasse gemäß EP-A-0 016 387 eingearbeitet:

| | |
|---|---|
| 0,2 Mol/l | Phosphatpuffer, pH 5,5 |
| 0,2 mMol/l | Substanz aus Beispiel 3 |
| 1,0 mMol/l | Anilinmethanphosphonsäure (gemäß EP-A-0 175 250) |
| 1,0 KU/l | Glucose-Oxidase |
| 10,0 KU/l | Peroxidase. |

Die Filmmasse wird mit einer Schichtdicke von 150 µm auf Papier als einen porösen Träger aufgerakelt. Zur Filmbildung wird die Masse ca. 30 Minuten bei 50 °C trocknen gelassen.

Zur Bestimmung der Glucosekonzentration (0 bis ca. 35 mmol/1) aus auf den Film aufgetragenem Serum bzw. Vollblut wird nach ca. 1 Minute umseitig mittels Photometer die Remission gemessen. Wie die nachstehende Wertetabelle erkennen läßt, ist eine präzise Ermittlung der Glucosekonzentration über eine vorher erstellte Calibrationskurve gut durchführbar.

Wertetabelle

| Glucose (mg/dl) | Remission (%) |
|---|---|
| 0 | 93 |
| 11 | 91 |
| 103 | 71 |
| 205 | 51 |
| 302 | 39 |
| 400 | 31 |
| 506 | 25 |
| 598 | 21 |

Beispiel 36

Bestimmung von Wasserstoffperoxid in wässriger Lösung

Reagenz-Lösungen:

| | |
|---|---|
| 1,75 mMol/l | Substanz aus Beispiel 19 |
| 2,0 mMol/l | Anilinmethanphosphonsäure (gemäß EP-A-0 175 250) |
| 1,25 KU/l | Peroxidase (Aktivitätsbestimmung mit Tetramethylbenzidin) |
| 100 mMol/l | Phosphatpuffer, pH 5,5 |

Zur Erstellung einer Kalibrationskurve werden in 1 cm-Küvetten 2,28 ml Phosphatpuffer vorgelegt und jeweils 100 µl Substanz aus Beispiel 22 und Anilinomethanphosphonsäure sowie 10 µl Peroxidase-Lösung zupipettiert. Die Probelösung erhält man durch Zugabe von 10 µl einer definierten Wasserstoffperoxid-Lösung. Die Referenzlösung ergibt sich durch Zugabe des gleichen Volumens Phosphatpuffer. Jeweils 30 Sekunden nach Wasserstoffperoxid-Zugabe wird die Extinktion des durch oxidative Kupplung gebildeten Farbstoffs als Funktion der eingesetzten Menge Peroxid bei $\lambda_{max}$ = 660 nm an einem Uvikon-Gerät bei 25 °C gemessen.

Die in nachstehender Tabelle zusammengefaßten Werte gehorchen über den gesamten Meßbereich dem Lambert-Beer'schen Gesetz. Der Korrelationskoeffizient der Regressionsgeraden beträgt 0,99985. Die eingesetzten $H_2O_2$-Lösungen entsprechen Glucose-Konzentrationen im diagnostisch relevanten Bereich von 30 mg/dl bis 600 mg/dl.

Tabelle:

| Extinktionswerte des Kupplungsproduktes als Funktion der $H_2O_2$-Konzentration | |
|---|---|
| mMol/l $H_2O_2$ | Extinktion |
| 1,74 | 0,132 |
| 3,48 | 0,232 |
| 6,96 | 0,448 |
| 10,44 | 0,622 |
| 13,92 | 0,858 |
| 17,40 | 1,051 |
| 20,88 | 1,253 |
| 24,36 | 1,463 |
| 27,84 | 1,635 |
| 31,32 | 1,842 |
| 34,80 | 2,061 |

## Patentansprüche

1. Verwendung eines Pyrazoloderivats gemäß der allgemeinen Formel I

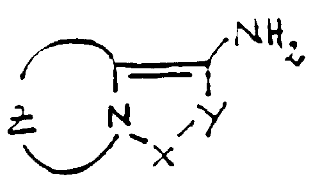

(I)

in der
X-Y $NR^1$-CO oder $N=CR^2$ bedeutet,
wobei

$R^1$ Alkyl und

$R^2$ Alkyl, Alkenyl, Alkoxy, Alkylthio, Aryl, Aralkyl, gegebenenfalls jeweils substituiert durch Hydroxy, Dialkylp-

hosphinyl, Carboxy, $SO_3H$, $PO_3H_2$, ein Salz eines dieser Säurereste oder/und Alkoxycarbonyl;

Amino, das gegebenenfalls durch ein oder zwei gegebenenfalls einen oder mehrere Hydroxy-, Carboxy- oder/ und Alkoxycarbonylreste tragende Alkylreste substituiert ist wobei, wenn Amino durch 2 Alkylreste stubstituiert ist, diese Reste auch zu einem Ring geschlossen sein können, der außer durch das N-Atom der Aminogruppe gegebenenfalls auch durch Sauerstoff, Schwefel oder ein weiteres Stickstoffatom unterbrochen sein kann, oder Amino gegebenenfalls durch ein oder zwei Acylgruppen, Alkoxy- oder/und Aralkoxycarbonylgruppen, $H_2N$-CO, Alkyl-, Aralkyl- oder/und Arylcarbamoylgruppen substituiert ist; oder

Wasserstoff, Carboxy, Alkoxycarbonyl, Carboxamido oder Halogen ist, und

Z $NR^3$-N=N bedeutet,

wobei $R^3$ Alkyl oder Aralkyl ist oder

Z eine ungesättigte Kette mit 3 bis 5 Gliedern aus Stickstoffatomen oder aus Kohlenstoffatomen und gegebenenfalls einem oder mehreren Stickstoff- oder Schwefelatomen darstellt, wobei Kohlenstoffatome gegebenenfalls substituiert sind durch Alkyl, Alkoxy, Alkylthio, Hydroxy, Aralkyl, Aryl, Carboxy, Carboxamido, Alkoxycarbonyl, Cyano, Amino, das gegebenenfalls durch ein oder zwei gegebenenfalls einen oder mehrere Hydroxy-, Carboxy- oder/und Alkoxycarbonylreste tragende Alkylreste substituiert ist oder Halogen und wobei Stickstoffatome, die nicht über eine Doppelbindung gebunden sind, durch Alkyl oder Aralkyl substituiert sind oder zwei benachbarte Kettensubstituenten gegebenenfalls eine Alkylengruppe bilden, die ihrerseits gegebenenfalls mit Aryl substituiert oder anelliert ist

wobei Alkyl und Alkoxy jeweils 1-6 C-Atome und Aryl 6-10 Ringatome umfaßt sowie gegebenenfalls entsprechende tautomere Formen und deren Salze

zur kolorimetrischen Bestimmung von Wasserstoffperoxid, wasserstoffperoxidbildenden Systemen, Peroxidase, peroxidatisch wirksamen Substanzen oder elektronenreichen aromatischen Verbindungen.

**2.** Verwendung eines Pyrazoloderivates gemäß Anspruch 1, dadurch gekennzeichnet, daß Z mindestens eine Doppelbindung enthält, die in Konjugation zu der Doppelbindung oder zu dem N-Atom der allgemeinen Formel I steht.

**3.** Verwendung eines Pyrazoloderivates gemäß einem der Ansprüche 1 und 2, dadurch gekennzeichnet, daß es aus der Gruppe der Substanzen der allgemeinen Formeln II bis XIII

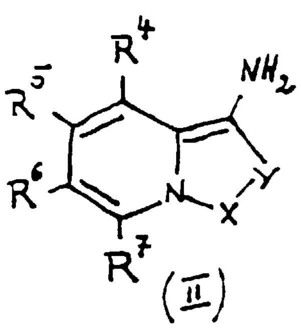
(II)

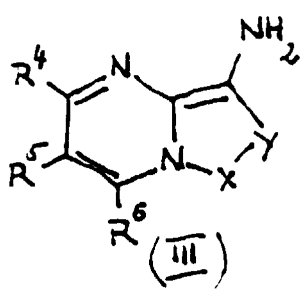
(III)

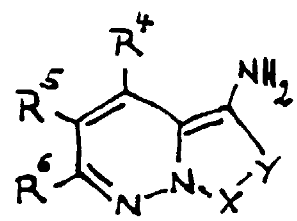
(IV)

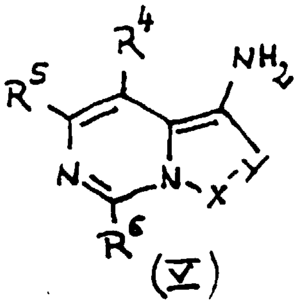

(V)

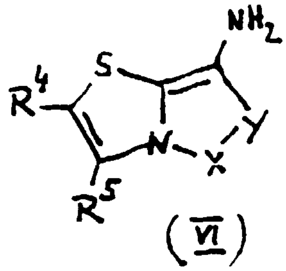

(VI)

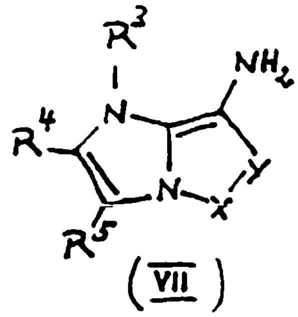

(VII)

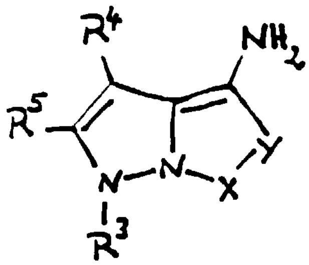

(VIII)

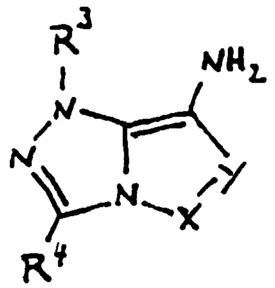

(IX)

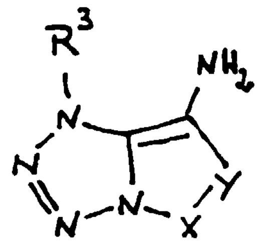

(X)

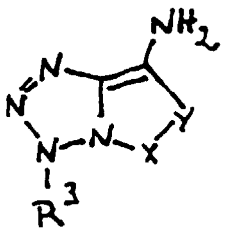

(XI)

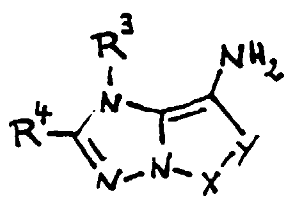

(XII)

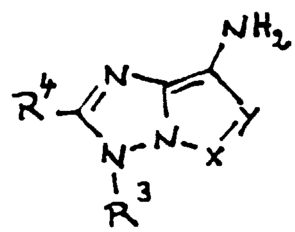

(XIII)

wobei X-Y und $R^3$ die gleiche Bedeutung wie in Anspruch 1 haben und

$R^4$, $R^5$, $R^6$ und $R^7$ gleich oder verschieden sind und Wasserstoff, Hydroxy, Alkyl, Alkoxy, Alkylthio, Aralkyl, Aryl, Carboxy, Alkoxycarbonyl, Carboxamido, Cyano, Amino, das gegebenenfalls durch ein oder zwei gegebenenfalls einen oder mehrere Hydroxy-, Carboxy- oder/und Alkoxycarbonylreste tragende Alkylreste substituiert ist oder Halogen bedeuten oder zwei benachbarte Reste gegebenenfalls eine Alkylengruppe bilden, die ihrerseits gegebenenfalls mit Aryl substituiert oder anelliert ist, sowie gegebenenfalls entsprechende tautomere Formen und deren Salze

gewählt ist.

**4.** Verwendung eines Pyrazoloderivates gemäß einem der Ansprüche 1 bis 3, dadurch gekennzeichnet, daß es aus der Gruppe der Substanzen der allgemeinen Formel II, III, IV, VI, VII und IX sowie gegebenenfalls entsprechende tautomere Formen und deren Salze gewählt ist.

**5.** Verwendung eines Pyrazoloderivates gemäß einem der Ansprüche 1 bis 4, dadurch gekennzeichnet, daß X-Y N=$CR^2$ bedeutet, wobei

$R^2$ die Bedeutung wie in Anspruch 1 hat.

**6.** Verfahren zur kolorimetrischen Bestimmung von Wasserstoffperoxid, Wasserstoffperoxid bildenden Systemen, Peroxidase oder peroxidatisch wirksamen Substanzen mittels oxidativer Kupplung einer elektronenreichen aromatischen Verbindung mit einer heterocyclischen Verbindung, dadurch gekennzeichnet, daß als heterocyclische Verbindung ein Pyrazoloderivat gemäß einem der Ansprüche 1 bis 5 eingesetzt wird.

**7.** Verfahren gemäß Anspruch 6, dadurch gekennzeichnet, daß als elektronenreiche aromatische Verbindung eine Substanz aus der Gruppe der Verbindungen, die mit p-Phenylendiamin eine oxidative Kupplung einzugehen vermögen, eingesetzt wird.

**8.** Mittel zur kolorimetrischen Bestimmung von Wasserstoffperoxid, Wasserstoffperoxid bildenden Systemen, Peroxidase oder peroxidatisch wirksamen Substanzen durch oxidative Kupplung enthaltend eine elektronenreiche aromatische Verbindung und eine heterocyclische Verbindung, dadurch gekennzeichnet, daß die heterocyclische Verbindung ein Pyrazoloderivat gemäß einem der Ansprüche 1 bis 5 ist.

**9.** Mittel gemäß Anspruch 8, dadurch gekennzeichnet, daß die elektronenreiche aromatische Verbindung eine Substanz aus der Gruppe der Verbindungen ist, die mit p-Phenylendiamin eine oxidative Kupplung einzugehen vermögen.

**10.** Verwendung eines Pyrazoloderivates gemäß einem der Ansprüche 1 bis 5 zur Herstellung eines Mittels zur kolorimetrischen Bestimmung von Wasserstoffperoxid, Wasserstoffperoxid bildenden Systemen, Peroxidase oder peroxidatisch wirksamen Substanzen durch oxidative Kupplung.

**11.** Verfahren zur kolorimetrischen Bestimmung einer elektronenreichen aromatischen Verbindung durch deren oxidative Kupplung mit einer heterocyclischen Verbindung in Gegenwart eines Oxidationsmittels, dadurch gekennzeichnet, daß als heterocyclische Verbindung ein Pyrazoloderivat gemäß einem der Ansprüche 1 - 5 eingesetzt wird.

**12.** Mittel zur kolorimetrischen Bestimmung einer elektronenreichen aromatischen Verbindung durch oxidative Kupplung enthaltend eine heterocyclische Verbindung und ein Oxidationsmittel, dadurch gekennzeichnet, daß die heterocyclische Verbindung ein Pyrazoloderivat gemäß einem der Ansprüche 1 - 5 ist.

**13.** Verwendung eines Pyrazoloderivates gemäß einem der Ansprüche 1 - 5 zur Herstellung eines Mittels zur kolorimetrischen Bestimmung einer elektronenreichen aromatischen Verbindung durch oxidative Kupplung.

**14.** Pyrazoloderivat der allgemeinen Formel I'

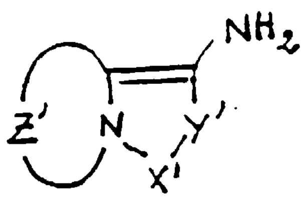

(I')

in der
X'-Y' $NR^{1'}$-CO oder $N=CR^{2'}$ bedeutet,
wobei

$R^{1'}$ Alkyl und
$R^{2'}$ Alkyl, Alkenyl, Alkoxy, Alkylthio, Aryl, Aralkyl, gegebenenfalls jeweils substituiert durch Hydroxy, Dialkylphosphinyl, Carboxy, $SO_3H$, $PO_3H_2$, ein Salz eines dieser Säurereste oder/und Alkoxycarbonyl;

Amino, das gegebenenfalls durch ein oder zwei gegebenenfalls einen oder mehrere Hydroxy-, Carboxy- oder/ und Alkoxycarbonylreste tragende Alkylreste substituiert ist wobei, wenn Amino durch 2 Alkylreste substituiert ist, diese Reste auch zu einem Ring geschlossen sein können, der außer durch das N-Atom der Aminogruppe gegebenenfalls auch durch Sauerstoff, Schwefel oder ein weiteres Stickstoffatom unterbrochen sein kann,

oder Amino gegebenenfalls durch ein oder zwei Acylgruppen, Alkoxy- oder/und Aralkoxycarbonylgruppen,

$H_2N$-CO, Alkyl-, Aralkyl- oder/und Arylcarbamoylgruppen substituiert ist; oder

Wasserstoff, Carboxy, Alkoxycarbonyl, Carboxamido oder Halogen ist, und

Z' $NR^{3'}$-N=N bedeutet,
wobei $R^{3'}$ Alkyl oder Aralkyl ist oder
Z' eine ungesättigte Kette mit 3 bis 5 Gliedern aus Stickstoffatomen oder aus Kohlenstoffatomen und gegebenenfalls einem oder mehreren Stickstoff- oder Schwefelatomen darstellt, wobei sämtliche Kohlenstoffatome der ungesättigten Kette Teil einer Doppelbindung sind und wobei Kohlenstoffatome gegebenenfalls substituiert sind durch Alkyl, Alkoxy, Alkylthio, Hydroxy, Aralkyl, Aryl, Carboxy, Carboxamido, Alkoxycarbonyl, Cyano, Amino, das gegebenenfalls durch ein oder zwei gegebenenfalls einen oder mehrere Hydroxy-, Carboxy- oder/und Alkoxycarbonylreste tragende Alkylreste substituiert ist oder Halogen und wobei Stickstoffatome, die nicht über eine Doppelbindung gebunden sind durch Alkyl oder Aralkyl substituiert sind oder zwei benachbarte Kettensubstituenten gegebenenfalls eine Alkylengruppe bilden, die ihrerseits gegebenenfalls mit Aryl substituiert oder anelliert ist, sowie gegebenenfalls ein entsprechendes Tautomer und dessen Salz
wobei Alkyl und Alkoxy jeweils 1-6 C-Atome und Aryl 6-10 Ringatome umfaßt unter der Bedingung, daß,

a) wenn X'-Y' gleich N=$CR^{2'}$ mit $R^{2'}$ gleich Alkyl oder Phenyl und
Z' eine ungesättigte Kette aus 4 Kohlenstoffatomen ist, diese nicht unsubstituiert und nicht durch Alkyl, Alkoxy oder Halogen substituiert ist,
b) wenn Z' eine ungesättigte Kette aus vier Gliedern mit 3 Kohlenstoffatomen und einem Stickstoffatom am Ende der Kette bedeutet, so daß in Formel I' der Ring mit Z' einen Pyrimidinring bildet und ein oder mehrere Kohlenstoffatome substituiert sind durch Alkyl, Alkoxy, Alkylthio, Hydroxy, Aryl, Alkoxycarbonyl oder Halogen oder mit einem Benzolring kondensiert ist und
X'-Y' für N=$CR^{2'}$ steht
$R^{2'}$ nicht Wasserstoff bedeutet,
c) wenn Z'

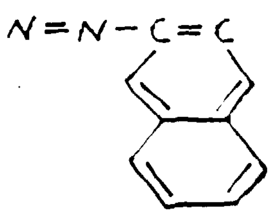

oder

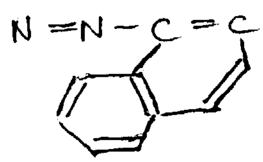

bedeutet, so daß in Formel I' der Ring mit Z' einen 1,2,4-Triazinring bildet und
X'-Y' für N=$CR^{2'}$ steht,
$R^{2'}$ nicht Methyl ist.

**15.** Pyrazoloderivat gemäß Anspruch 14, dadurch gekennzeichnet, daß Z' mindestens eine Doppelbindung enthält, die in Konjugation zu der Doppelbindung oder zu dem N-Atom der allgemeinen Formel I' steht.

**16.** Pyrazoloderivat gemäß einem der Ansprüche 14 und 15, dadurch gekennzeichnet, daß es aus der Gruppe der Substanzen der allgemeinen Formel II' bis XIII'

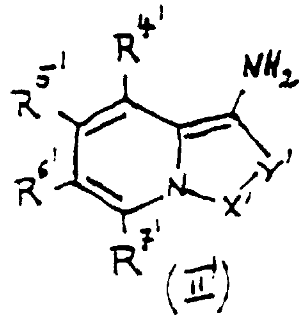

(II')

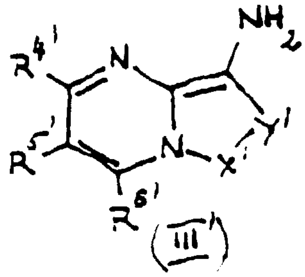

(III')

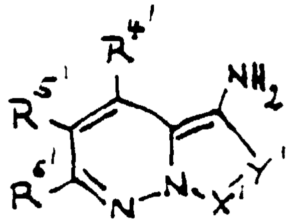

(IV')

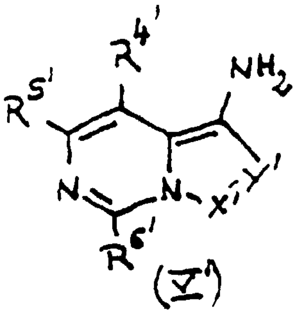

(V')

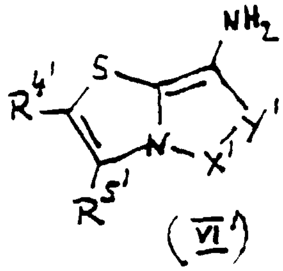

(VI')

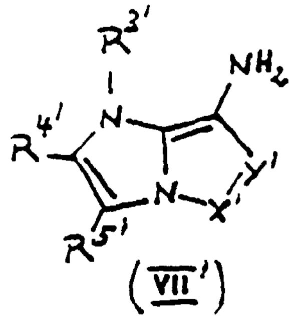

(VII')

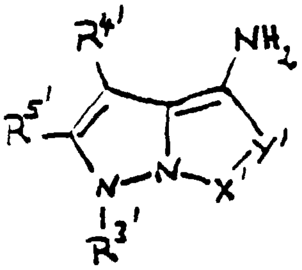

(VIII')

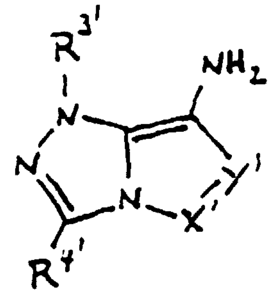

(IX')

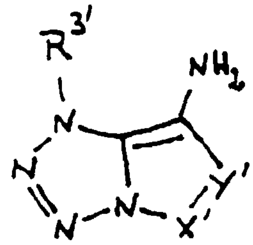

(X')

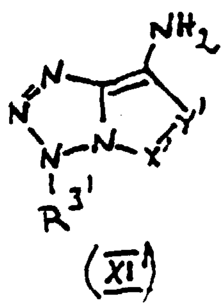

(XI')

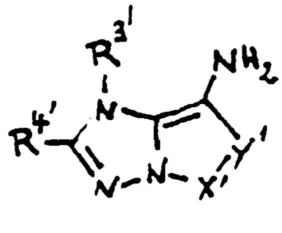

(XII')

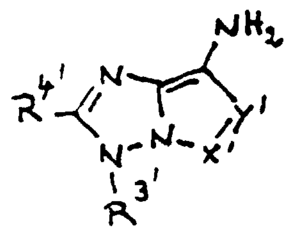

(XIII')

wobei

X'-Y' und $R^{3'}$ die gleiche Bedeutung wie in Anspruch 14 haben und
$R^{4'}$, $R^{5'}$, $R^{6'}$ und $R^{7'}$ gleich oder verschieden sind und Wasserstoff, Hydroxy, Alkyl, Alkoxy, Alkylthio, Aralkyl,

Aryl, Carboxy, Alkoxycarbonyl, Carboxamido, Cyano, Amino, das gegebenenfalls durch ein oder zwei gegebenenfalls einen oder mehrere Hydroxy-, Carboxy- oder/und Alkoxycarbonylreste tragende Alkylreste substituiert ist oder Halogen bedeuten oder zwei benachbarte Reste gegebenenfalls eine Alkylengruppe bilden, die ihrerseits gegebenenfalls mit Aryl substituiert oder anelliert ist,

sowie gegebenenfalls ein entsprechendes Tautomer und dessen Salz

gewählt ist.

**17.** Pyrazoloderivat gemäß einem der Ansprüche 14 bis 16, dadurch gekennzeichnet, daß es aus der Gruppe der Substanzen der allgemeinen Formeln II', III', IV', VI', VII' und IX', sowie gegebenenfalls entsprechende tautomere Formen und deren Salze gewählt ist.

**18.** Pyrazoloderivat gemäß einem der Ansprüche 14 bis 17, dadurch gekennzeichnet, daß
X'-Y' $N=CR^{2'}$ bedeutet, wobei
$R^{2'}$ die Bedeutung wie in Anspruch 14 hat.

**19.** Verfahren zur Herstellung von Pyrazoloderivaten gemäß Anspruch 14, dadurch gekennzeichnet, daß eine Verbindung der allgemeinen Formel XIV

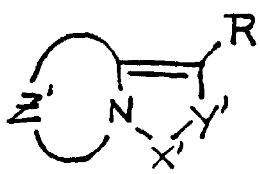

(XIV)

in der
X'-Y' und Z' die gleiche Bedeutung wie in Anspruch 14 haben und
R Wasserstoff ist,

a) durch Umsetzung mit Salpetersäure oder Salpetersäure im Gemisch mit Schwefelsäure und/oder Acetanhydrid in eine Verbindung der allgemeinen Formel XIV mit R gleich Nitro oder

b) durch Reaktion mit salpetriger Säure in eine Verbindung der algemeinen Formel XIV mit R gleich Nitroso oder

c) durch Reaktion mit einem aromatischen Diazoniumsalz in eine Verbindung der allgemeinen Formel XIV mit R gleich Arylazo

überführt und anschließend reduziert wird.

## Claims

**1.** Use of a pyrazolo derivative of the general formula I

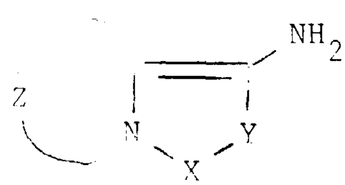

(I)

in which X-Y signifies $NR^1$-CO or $N=CR^2$, whereby $R^1$ is alkyl and $R^2$ alkyl, alkenyl, alkoxy, alkylthio, aryl, aralkyl, in each case possibly substituted by hydroxyl, dialkylphosphinyl, carboxyl, $SO_3H$, $PO_3H_2$, a salt of one of these

acid residues and/or alkoxycarbonyl; amino, which is possibly substituted by one or two alkyl radicals possibly carrying one or more hydroxyl, carboxyl and/or alkoxycarbonyl radicals, whereby, when amino is substituted by 2 alkyl radicals, these radicals can also be joined to form a ring which, apart from by the N-atom of the amino group, can possibly also be interrupted by oxygen, sulphur or a further nitrogen atom, or amino is possibly substituted by one or two acyl groups, alkoxy- and/or aralkoxycarbonyl groups, $H_2N$-CO-, alkyl-, aralkyl- and/or arylcarbamoyl groups; or is hydrogen, carboxyl, alkoxycarbonyl, carboxamido or halogen and Z signifies $NR^3$-N=N, whereby $R^3$ is alkyl or aralkyl or Z represents an unsaturated chain with 3 to 5 members from nitrogen atoms or from carbon atoms and possibly one or more nitrogen or sulphur atoms, whereby carbon atoms are possibly substituted by alkyl, alkoxy, alkylthio, hydroxyl, aralkyl, aryl, carboxyl, carboxamido, alkoxycarbonyl, cyano, amino, which is possibly substituted by one or two alkyl radicals optionally carrying one or more hydroxyl, carboxyl and/or alkoxycarbonyl radicals, or halogen, whereby nitrogen atoms which are not connected via a double bond are substituted by alkyl or aralkyl or two neighbouring chain substituents possibly form an alkylene radical which, in turn, is possibly substituted or anellated with aryl, whereby alkyl and alkoxy each include 1 - 6 C-atoms and aryl 6 - 10 ring atoms, as well as possibly corresponding tautomeric forms and their salts for the colorimetric determination of hydrogen peroxide, hydrogen peroxide-forming systems, peroxidase, peroxidate-active substances or electron-rich aromatic compounds.

**2.** Use of a pyrazolo derivative according to claim 1, characterised in that Z contains at least one double bond which stands in conjunction with the double bond or with the N-atom of the general formula I.

**3.** Use of a pyrazolo derivative according to one of claims 1 and 2, characterised in that it is selected from the group of the substances of the general formulae II to XIII:

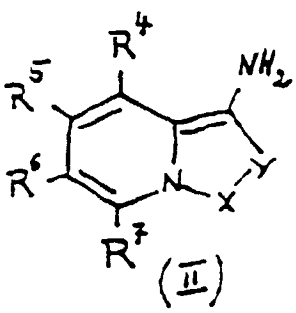
(II)

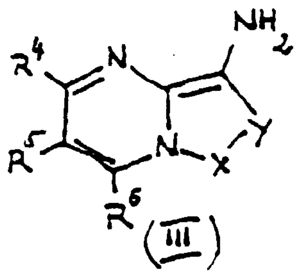
(III)

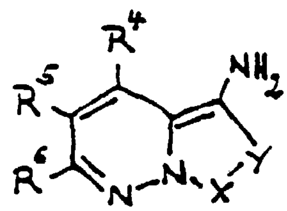
(IV)

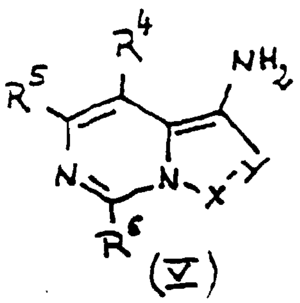
(V)

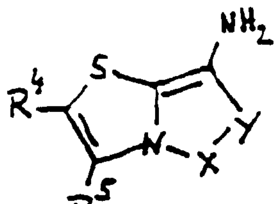
(VI)

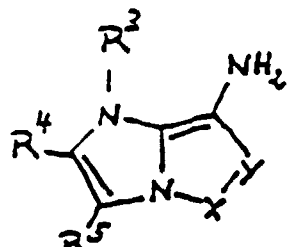
(VII)

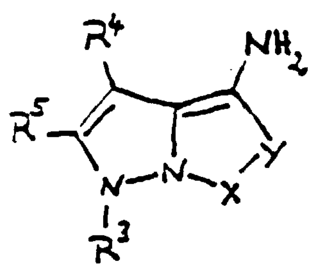

(VIII)

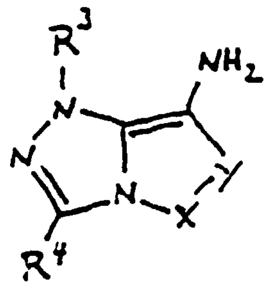

(IX)

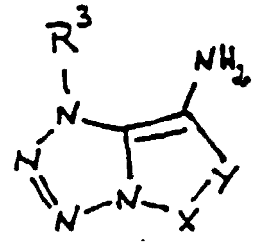

(X)

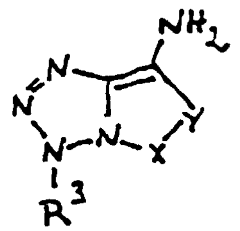

(XI)

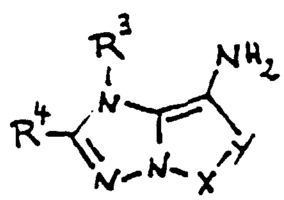

(XII)

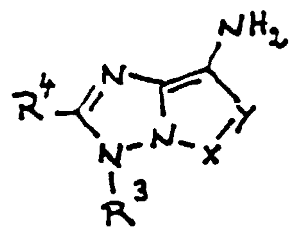

(XIII)

whereby X-Y and $R^3$ have the same meaning as in claim 1 and $R^4$, $R^5$, $R^6$ and $R^7$ are the same or different and signify hydrogen, hydroxyl, alkyl, alkoxy, alkylthio, aralkyl, aryl, carboxyl, alkoxycarbonyl, carboxamido, cyano, amino, which is possibly substituted by one or two alkyl radicals possibly carrying one or more hydroxyl, carboxyl and/or alkoxycarbonyl radicals, or halogen or two neighbouring radicals possibly form an alkylene group which, in turn, is possibly substituted or anellated with aryl, as well as possibly corresponding tautomeric forms and their salts.

**4.** Use of a pyrazolo derivative according to one of claims 1 to 3, characterised in that it is selected from the group of the substances of the general formulae II, III, IV, VI, VII and IX, as well as possibly corresponding tautomeric forms and their salts.

**5.** Use of a pyrazolo derivative according to one of claims 1 to 4, characterised in that X-Y signifies $N=CR^2$, whereby $R^2$ has the meaning as in claim 1.

**6.** Process for the colorimetric determination of hydrogen peroxide, hydrogen peroxide-forming systems, peroxidase or peroxidate-active substances by means of oxidative coupling of an electron-rich aromatic compound with a heterocyclic compound, characterised in that, as heterocyclic compound, a pyrazolo derivative according to one of claims 1 to 5 is used.

**7.** Process according to claim 6, characterised in that, as electron-rich aromatic compound, a substance is used from the group of compounds which are able to enter into an oxidative coupling with p-phenylenediamine.

**8.** Agent for the colorimetric determination of hydrogen peroxide, hydrogen peroxide-forming systems, peroxidase or peroxidate-active substances by oxidative coupling, containing an electron-rich aromatic compound and a heterocyclic compound, characterised in that the heterocyclic compound is a pyrazolo derivative according to one of claims 1 to 5.

**9.** Agent according to claim 8, characterised in that the electron-rich aromatic compound is a substance from the group of compounds which are able to enter into an oxidative coupling with p-phenylenediamine.

**10.** Use of a pyrazolo derivative according to one of claims 1 to 5 for the preparation of an agent for the colorimetric determination of hydrogen peroxide, hydrogen peroxide-forming systems, peroxidase or peroxidate-active substances by oxidative coupling.

**11.** Process for the colorimetric determination of an electron-rich aromatic compound by its oxidative coupling with a heterocyclic compound in the presence of an oxidation agent, characterised in that, as heterocyclic compound, a pyrazolo derivative according to one of claims 1 - 5 is used.

**12.** Agent for the colorimetric determination of an electron-rich aromatic compound by oxidative coupling, containing a heterocyclic compound and an oxidation agent, characterised in that the heterocyclic compound is a pyrazolo derivative according to one of claims 1 - 5.

**13.** Use of a pyrazolo derivative according to one of claims 1 - 5 for the preparation of an agent for the colorimetric determination of an electron-rich aromatic compound by oxidative coupling.

**14.** Pyrazolo derivative of the general formula I'

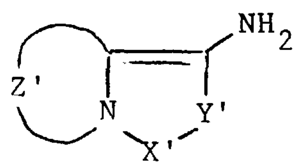

(I')

in which X'-Y' signifies $NR^{1'}$-CO or $N=CR^{2'}$, whereby $R^{1'}$ is alkyl and $R^{2'}$ alkyl, alkenyl, alkoxy, alkylthio, aryl, aralkyl, possibly in each case substituted by hydroxyl, dialkylphosphinyl, carboxy, $SO_3H$, $PO_3H_2$, a salt of one of these acid residues and/or alkoxycarbonyl; amino, which is possibly substituted by one or two alkyl radicals possibly carrying one or more hydroxyl, carboxyl and/or alkoxycarbonyl radicals, whereby, when amino is substituted by 2 alkyl radicals, these radicals can be joined to form a ring which, apart from the N-atom of the amino group, can possibly also be interrupted by oxygen, sulphur or a further nitrogen atom, or amino is possibly substituted by one or two acyl groups, alkoxy- and/or aralkoxycarbonyl groups, $H_2N$-CO, alkyl-, aralkyl- and/or arylcarbamoyl groups; or is hydrogen, carboxyl, alkoxycarbonyl, carboxamido or halogen, and Z' signifies $NR^{3'}$-N=N, whereby $R^{3'}$ is alkyl or aralkyl or Z' represents an unsaturated chain with 3 to 5 members from nitrogen atoms or from carbon atoms and possibly one or more nitrogen or sulphur atoms, whereby all carbon atoms of the unsaturated chain are part of a double bond and whereby carbon atoms are possibly substituted by alkyl, alkoxy, alkylthio, hydroxyl, aralkyl, aryl, carboxyl, carboxamido, alkoxycarbonyl, cyano, amino, which is possibly substituted by one or two alkyl radicals possibly carrying one or more hydroxyl, carboxyl and/or alkoxycarbonyl radicals, or halogen and whereby nitrogen atoms which are not bound by a double bond are substituted by alkyl or aralkyl or two neighbouring chain substituents possibly form an alkylene group which, in turn, is possibly substituted or anellated with aryl, as well as possibly a corresponding tautomer and its salts, whereby alkyl and alkoxy in each case include 1 - 6 C-atoms and aryl 6 - 10 ring atoms, with the proviso that

a) when X'-Y' is $N=CR^{2'}$ with $R^{2'}$ being alkyl or phenyl and Z' is an unsaturated chain of 4 carbon atoms, this is not unsubstituted and not substituted by alkyl, alkoxy or halogen,
b) when Z' signifies an unsaturated chain of four members with 3 carbon atoms and one nitrogen atom on the end of the chain, so that in formula I' the ring with Z' forms a pyrimidine ring and one or more carbon atoms are substituted by alkyl, alkoxy, alkylthio, hydroxyl, aryl, alkoxycarbonyl or halogen or is condensed with a benzene ring and X'-Y' stands for $N=CR^{2'}$, $R^{2'}$ does not signify hydrogen,
c) when Z' signifies

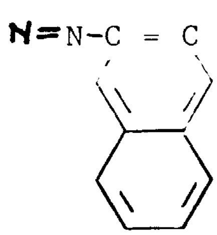

or

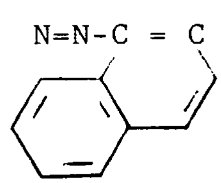

so that in formula I' the ring with Z' forms a 1,2,4-triazine ring and X'-Y' stands for N=C$R^{2'}$, $R^{2'}$ is not methyl.

**15.** Pyrazolo derivative according to claim 14, characterised in that Z' contains at least one double bond which stands in conjunction with the double bond or with the N-atom of general formula I'.

**16.** Pyrazolo derivative according to one of claims 14 and 15, characterised in that it is selected from the group of substances of the general formulae II' to XIII':

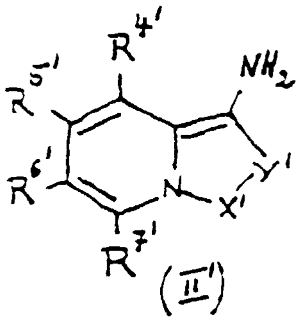

(II')

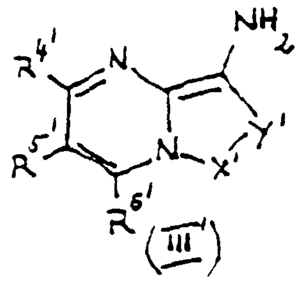

(III')

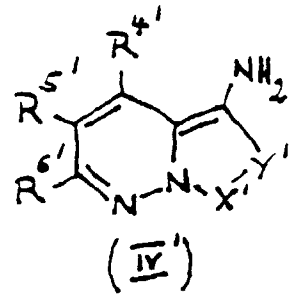

(IV')

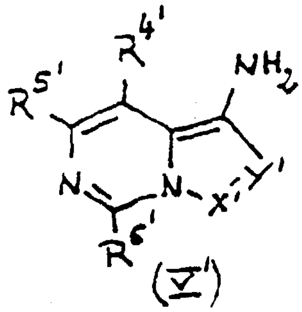

(V')

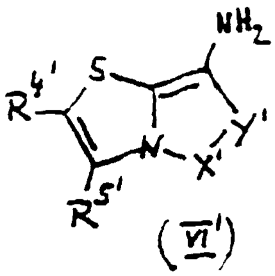

(VI')

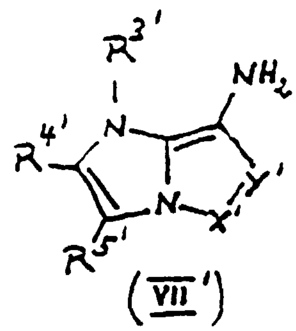

(VII')

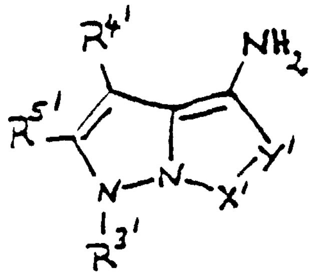

(VIII')

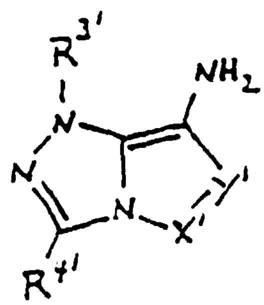

(IX')

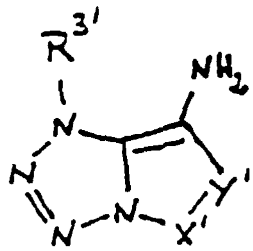

(X')

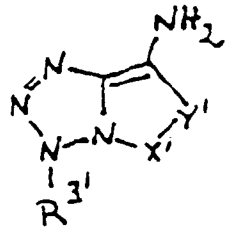

(XI')

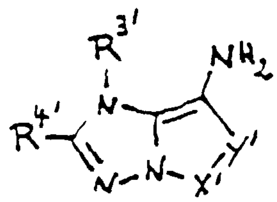

(XII')

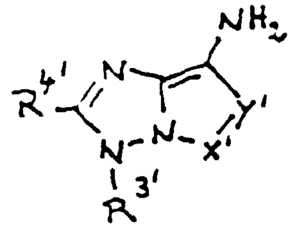

(XIII')

whereby X'-Y' and $R^{3'}$ have the same meaning as in claim 14 and $R^{4'}$, $R^{5'}$, $R^{6'}$ and $R^{7'}$ are the same or different and signify hydrogen, hydroxyl, alkyl, alkoxy, alkylthio, aralkyl, aryl, carboxyl, alkoxycarbonyl, carboxamido, cyano, amino which is possibly substituted by one or two alkyl radicals possibly carrying one or more hydroxyl, carboxyl and/or alkoxycarbonyl radicals, or halogen or two neighbouring radicals possibly form an alkylene group which, in turn, is possibly substituted or anellated with aryl, as well as possibly a corresponding tautomer and its salts.

**17.** Pyrazolo derivative according to one of claims 14 to 16, characterised in that it is selected from a group of substances of the general formulae II', III', IV', VI', VII' and IX', as well as possibly corresponding tautomeric forms and its salts.

**18.** Pyrazolo derivative according to one of claims 14 to 17, characterised in that X'-Y' signifies N=$CR^{2'}$, whereby $R^{2'}$ has the meaning as in claim 17.

**19.** Process for the preparation of pyrazolo derivatives according to claim 14, characterised in that a compound of the general formula XIV

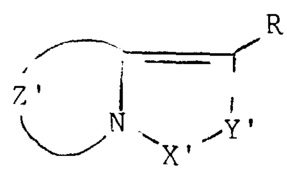

(XIV)

in which X'-Y' and Z' have the same meaning as in claim 14 and R is hydrogen, is converted

a) by reaction with nitric acid or nitric acid in admixture with sulphuric acid and/or acetic anhydride into a compound of the general formula XIV with R equal to nitro or
b) by reaction with nitrous acid into a compound of the general formula XIV with R equal to nitroso or
c) by reaction with an aromatic diazonium salt into a compound of general formula XIV with R equal to arylazo,

and subsequently reduced.

### Revendications

**1.** Utilisation d'un dérivé pyrazolo de formule générale I

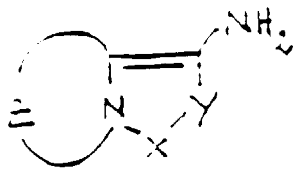

dans laquelle

X-Y représente $NR^1$-CO ou $N{=}CR^2$,

où

$R^1$ représente un groupe alkyle et

$R^2$ représente un groupe alkyle, alcényle. alcoxy, alkylthio, aryle, aralkyle, chacun des ces groupes étant éventuellement substitué par un groupe hydroxy, dialkylphosphinyle, carboxy, $SO_3H$, $PO_3H_2$, un sel de ces restes d'acides et/ou un groupe alcoxycarbonyle ;
un groupe amino, qui peut être éventuellement substitué par un ou deux restes alkyle portant éventuellement un ou plusieurs restes hydroxy, carboxy et/ou alcoxycarbonyle, étant entendu que dans le cas où le groupe amine est substitué par 2 restes alkyle, ces restes peuvent également former un cycle, qui peut être interrompu, en plus de l'atome N du groupe amino, éventuellement également par un atome d'oxygène, de soufre ou un autre atome d'azote, ou le groupe amino est éventuellement substitué par un ou deux groupes acyle, alcoxy et/ou aralcoxycarbonyle, $H_2N$-CO, alkyle, aralkyle et/ou arylcarbamoyle ; ou
un atome d'hydrogène, un groupe carboxy, alcoxycarbonyle, carboxamido ou halogéno, et

Z représente un groupe $NR^3$-N=N,

dans lequel $R^3$ représente un groupe alkyle ou aralkyle, ou

Z représente une chaîne insaturée ayant de 3 à 5 maillons formés d'atomes d'azote ou d'atomes de carbone, et éventuellement un ou plusieurs atomes d'azote ou de soufre, les atomes de carbone étant éventuellement substitués par un groupe alkyle, alcoxy, alkylthio, hydroxy, aralkyle, aryle, carboxy, carboxamido, alcoxycarbonyle, cyano, amino qui peut être éventuellement substitué par un ou deux restes alkyle portant éventuellement un ou plusieurs groupes hydroxy, carboxy et/ou alcoxycarbonyle, ou un atome d'halogène. et étant entendu que les atomes d'azote qui ne sont pas liés par une double liaison sont substitués par un groupe alkyle ou aralkyle, ou deux substituants contigus sur la chaîne forment éventuellement un groupe alkylène qui, à son tour, est éventuellement substitué par, ou annelle à, un groupe aryle,

où les groupes alkyle et alcoxy comprennent chacun de 1 à 6 atomes de C et les groupes aryle comprennent de 6 à 10 atomes de cycle,

ainsi qu'éventuellement les formes tautomères correspondantes et leurs sels,

pour la détermination colorimétrique du peroxyde d'hydrogène, de systèmes formant du peroxyde d'hydrogène, de peroxydases, de substances ayant une activité de peroxydase ou de composés aromatiques riches en électrons.

2. Utilisation d'un dérivé pyrazolo selon la revendication 1, caractérisée en ce que Z contient au moins une double liaison, qui est conjuguée à la double liaison ou à l'atome N de la formule générale I.

3. Utilisation d'un dérivé pyrazolo selon l'une des revendications 1 et 2, caractérisée en ce Que ledit dérivé est choisi dans le groupe des substances répondant aux formules générales II à XIII

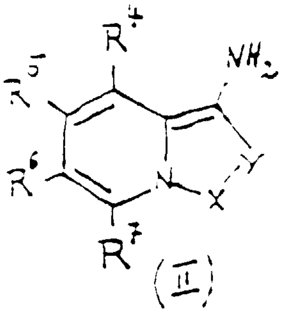

(II)

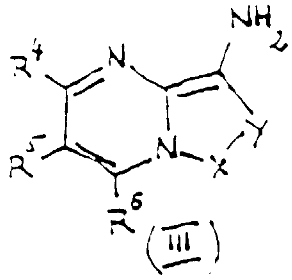

(III)

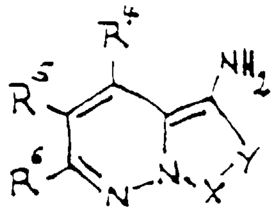

(IV)

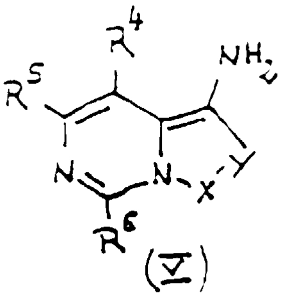

(V)

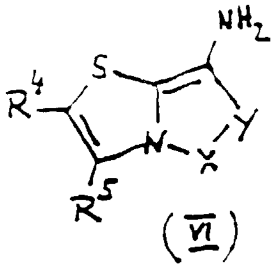

(VI)

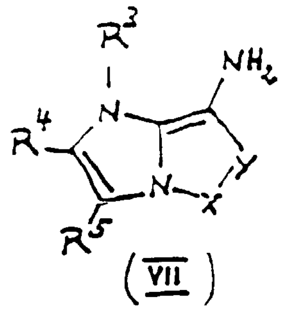

(VII)

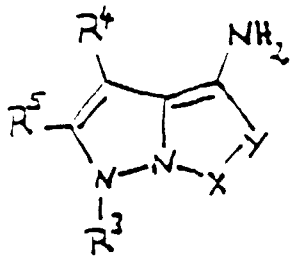

(VIII)

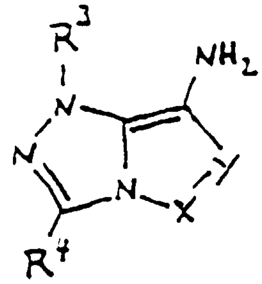

(IX)

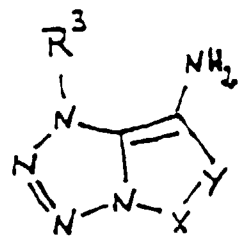

(X)

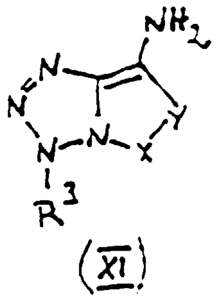

(XI)

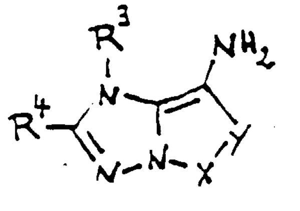

(XII)

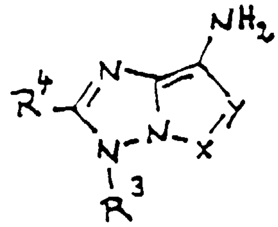

(XIII)

X-Y et $R^3$ ayant les mêmes significations que dans la revendication 1, et

$R^4$, $R^5$, $R^6$ et $R^7$ sont identiques ou différents et représentent un atome d'hydrogène, un groupe hydroxy, alkyle, alcoxy, alkylthio, aralkyle, aryle, carboxy, alcoxycarbonyle, carboxamido, cyano, amino pouvant être éventuellement substitué par un ou deux restes alkyle portant éventuellement un ou plusieurs groupes hydroxy, carboxy et/ou alcoxycarbonyle, ou un atome d'halogène, ou deux restes contigüs forment éventuellement un groupe alkylène qui, à son tour, est éventuellement substitué par, ou annellé à, un groupe aryle, ainsi qu'éventuellement leurs formes tautomères correspondantes et leurs sels.

**4.** Utilisation d'un dérivé pyrazolo selon l'une quelconque des revendications 1 à 3, caractérisée en ce que ledit dérivé est choisi dans le groupe des substances répondant aux formules générales II, III, IV, VI, VII et IX, ainsi qu'éventuellement leurs formes tautomères correspondantes et leurs sels.

**5.** Utilisation d'un dérivé pyrazolo selon l'une quelconque des revendications 1 à 4, caractérisée en ce que

X-Y représente $N=CR^2$,

où

$R^2$ à la même signification que dans la revendication 1.

**6.** Procédé de détermination colorimétrique du peroxyde d'hydrogène, de systèmes formant du peroxyde d'hydrogène, de peroxydases ou de substances ayant une activité de peroxydase, par couplage oxydatif d'un composé aromatique riche en électrons avec un composé hétérocyclique, caractérisé en ce que, en tant que composé hétérocyclique, on met en oeuvre un dérivé pyrazolo selon l'une quelconque des revendications 1 à 5.

**7.** Procédé selon la revendication 6, caractérisé en ce que, en tant que composé aromatique riche en électrons, on utilise une substance appartenant au groupe des composés qui permettent de réaliser un couplage oxydatif avec la p-phénylènediamine.

**8.** Agent destiné à la détermination colorimétrique du peroxyde d'hydrogène, de systèmes formant du peroxyde d'hydrogène, de peroxydases ou de substances ayant une activité de peroxydase, par couplage oxydatif d'un composé aromatique riche en électrons avec un composé hétérocyclique, caractérisé en ce que le composé hétérocyclique est un dérivé pyrazolo selon l'une quelconque des revendications 1 à 5.

**9.** Agent selon la revendication 8, caractérisé en ce que le composé aromatique riche en électrons est une substance appartenant au groupe des composés qui permettent de réaliser un couplage oxydatif avec la p-phénylènediamine.

**10.** Utilisation d'un dérivé pyrazolo selon l'une quelconque des revendications 1 à 5 pour la préparation d'un agent destiné à la détermination colorimétrique du peroxyde d'hydrogène, de systèmes formant du peroxyde d'hydrogène, de peroxydases ou de substances ayant une activité de peroxydase, par couplage oxydatif.

**11.** Procédé de détermination colorimétrique d'un composé aromatique riche en électrons, par couplage oxydatif de celui-ci à un composé hétérocyclique en présence d'un agent oxydant, caractérisé en ce que, en tant que composé hétérocyclique, on utilise un dérivé pyrazolo selon l'une quelconque des revendications 1 à 5.

**12.** Agent destiné à la détermination colorimétrique d'un composé aromatique riche en électrons, par couplage oxydatif, contenant un composé hétérocyclique et un agent oxydant, caractérisé en ce que le composé hétérocyclique est un dérivé pyrazolo selon l'une quelconque des revendications 1 à 5.

**13.** Utilisation d'un dérivé pyrazolo selon l'une quelconque des revendications 1 à 5, pour la préparation d'un agent destiné à la détermination colorimétrique d'un composé aromatique riche en électrons, par couplage oxydatif.

**14.** Dérivé pyrazolo de formule générale I'

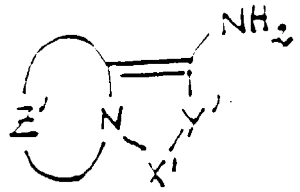

(I')

dans laquelle

X'-Y' représente $NR^{1'}$-CO ou $N=CR^{2'}$.

où

$R^{1'}$ représente un groupe alkyle et

$R^{2'}$ représente un groupe alkyle, alcényle, alcoxy, alkylthio, aryle, aralkyle, chacun des ces groupes étant éventuellement substitué par un groupe hydroxy, dialkylphosphinyle, carboxy, $SO_3H$, $PO_3H_2$, un sel de ces restes d'acides et/ou un groupe alcoxycarbonyle ;
un groupe amino, qui peut être éventuellement substitué par un ou deux restes alkyle portant éventuellement un ou plusieurs restes hydroxy, carboxy et/ou alcoxycarbonyle, étant entendu que dans le cas où le groupe amino est substitué par 2 restes alkyle, ces restes peuvent également former un cycle, qui peut être interrompu, en plus de l'atome N du groupe amino, éventuellement également par un atome d'oxygène, de soufre ou un autre atome d'azote, ou le groupe amino est éventuellement substitué par un
ou deux groupes acyle, alcoxy et/ou aralcoxycarbonyle $H_2N$-CO, alkyle, aralkyle et/ou arylcarbamoyle ; ou un atome d'hydrogène, un groupe carboxy, alcoxycarbonyle, carboxamido ou halogéno. et

Z' représente un groupe $NR^{3'}$-N=N,

dans lequel $R^{3'}$ représente un groupe alkyle ou aralkyle, ou
Z' représente une chaîne insaturée ayant de 3 à 5 maillons formés d'atomes d'azote ou d'atomes de carbone, et éventuellement un ou plusieurs atomes d'azote ou de soufre, tous les atomes de carbone de la partie de chaîne insaturée participant à une double liaison et les atomes de carbone étant éventuellement substitués par un groupe alkyle, alcoxy, alkylthio, hydroxy, aralkyle, aryle, carboxy, carboxamido, alcoxycarbonyle, cyano, amino pouvant être éventuellement substitué par un ou deux restes alkyle portant éventuellement un ou plusieurs groupes hydroxy, carboxy et/ou alcoxycarbonyle, ou un atome d'halogène, et étant entendu que les atomes d'azote qui ne sont pas liés par une double liaison sont substitués par un groupe alkyle ou aralkyle, ou deux substituants contigüs sur la chaîne forment éventuellement un groupe alkylène qui, à son tour, est éventuellement substitué par, ou annellé à, un groupe aryle, ainsi qu'éventuellement un tautomère correspondant et ses sels,
où les groupes alkyle et alcoxy comprennent chacun de 1 à 6 atomes de C et les groupes aryle comprennent de 6 à 10 atomes de cycle, étant entendu que

a) lorsque X'-Y' représente $N=CR^{2'}$, $R^{2'}$ étant un groupe alkyle ou phényle, et
Z' représente une chaîne insaturée comprenant 4 atomes de carbone, cette chaîne n'est pas non substituée et n'est pas substituée par un groupe alkyle, alcoxy ou halogéno,

b) lorsque Z' représente une chaîne insaturée comprenant quatre maillons constitués par 3 atomes de carbone et un atome d'azote à l'extrémité de la chaîne, de sorte que dans la formule I', le cycle avec Z' forme un cycle pyrimidine et un ou plusieurs atomes de carbone sont substitués par un groupe alkyle, alcoxy, alkylthio, hydroxy, aryle, alcoxycarbonyle ou halogéno, ou il est condensé à un cycle benzénique et
X'-Y' représente $N=CR^{2'}$,
$R^{2'}$ ne représente pas un atome d'hydrogène,

c) lorsque Z' représente

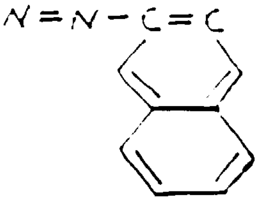

ou

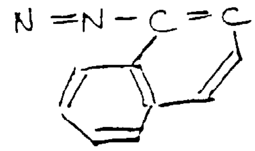

de sorte que dans la formule I', le cycle avec Z' forme un cycle 1,2,4-triazine et
X'-Y' représente N=CR$^{2'}$,
R$^{2'}$ ne représente pas un groupe méthyle.

**15.** Dérivé pyrazolo selon la revendication 14, caractérisé en ce que Z' contient au moins une double liaison qui est conjuguée à la double liaison ou à l'atome N de la formule générale I'.

**16.** Dérivé pyrazolo selon l'une quelconque des revendications 14 et 15, caractérisé en ce qu'il est choisi dans le groupe des substances répondant aux formules générales II' à XIII'

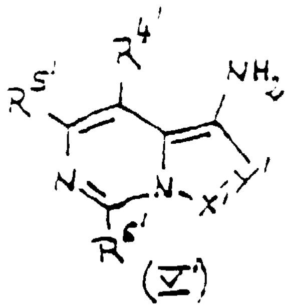

(V')

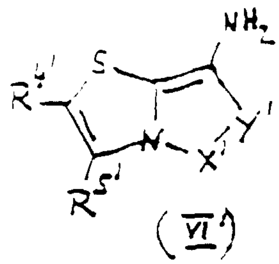

(VI')

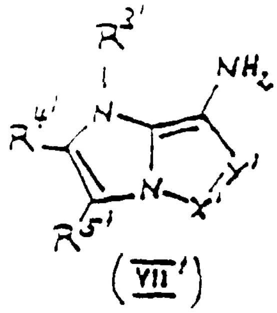

(VII')

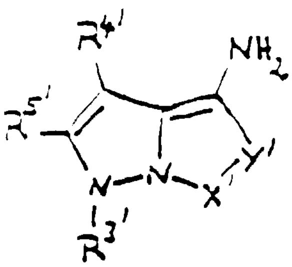

(VIII')

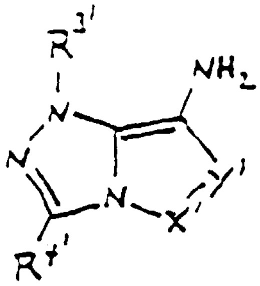

(IX')

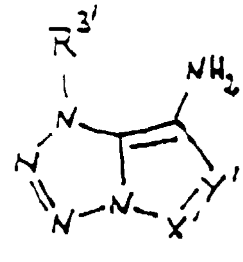

(X')

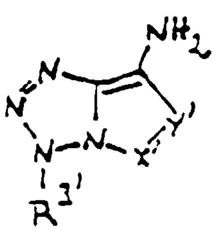

(XI')

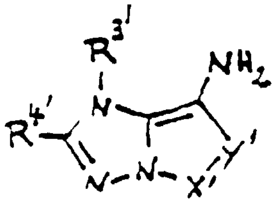

(XII')

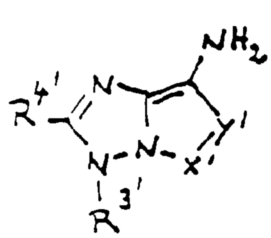

(XIII')

où

X'-Y' et $R^{3'}$ ont les mêmes significations que dans la revendication 14 et

$R^{4'}$, $R^{5'}$, $R^{6'}$ et $R^{7'}$ sont identiques ou différents et représentent un atome d'hydrogène, un groupe hydroxy, alkyle, alcoxy, alkylthio, aralkyle, aryle, carboxy, alcoxycarbonyle, carboxamido, cyano, amino pouvant être éventuellement substitué par un ou deux restes alkyle portant éventuellement un ou plusieurs groupes hydroxy, carboxy et/ou alcoxycarbonyle. ou un atome d'halogène, ou deux restes contigüs forment éventuellement un groupe alkylène qui, à son tour, est éventuellement substitué par, ou annellé à, un groupe aryle,

ainsi qu'éventuellement leurs formes tautomères correspondantes et leurs sels.

**17.** Dérivé pyrazolo selon l'une quelconque des revendications 14 à 16, caractérisé en ce qu'il est choisi dans le groupe des substances répondant aux formules générales II', III', IV', VI', VII' et IX', ainsi qu'éventuellement leurs formes tautomères correspondantes et leurs sels.

**18.** Dérivé pyrazolo selon l'une quelconque des revendications 14 à 17, caractérisé en ce que
X'-Y' représente N=$CR^{2'}$,
où $R^{2'}$ a la même signification que dans la revendication 14.

**19.** Procédé de préparation d'un dérivé pyrazolo selon la revendication 14, caractérisé en ce qu'un composé de formule générale XIV

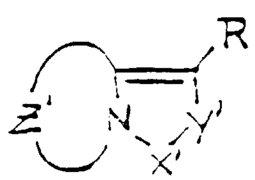

(XIV)

dans laquelle
X'-Y et Z' ont la même signification que dans la revendication 14,
et
R représente un atome d'hydrogène,
est transformé

a) par réaction avec de l'acide nitrique ou un mélange d'acide nitrique et d'acide sulfurique et/ou de l'anhydride acétique, en un composé de formule générale XIV, R représentant un groupe nitro, ou

b) par réaction avec de l'acide nitreux, en un composé de formule générale XIV, R représentant un groupe nitroso, ou

c) par réaction avec un sel de diazonium aromatique, en un composé de formule générale XIV, R représentant un groupe arylazo,

et ensuite, il est réduit.